# EUROPEAN PATENT APPLICATION

(11) **EP 3 550 019 A1**
(43) Date of publication of application: **09.10.2019**
(21) Application number: 19166017.4
(22) Date of filing: 20.10.2015
(51) Int. Cl.: C12N 15/11, C12N 15/113, A61K 31/7088

(54) **COMBINATION**

(30) Priority: 24.10.2014 US 201462068141 P
(62) Divisional of application: 15784628.8
(71) Applicant: Astrazeneca AB, 151 85 Södertälje (SE)
(72) Inventor: WOESSNER, Richard, Waltham, MA 02451 (US); MCCOON, Patricia, Elizabeth, Waltham, MA 02451 (US); LYNE, Paul, Dermot, Waltham, MA 02451 (US)
(74) Representative: AstraZeneca

(57) **Abstract**

The present invention features methods of treating cancer with an immunomodulatory agent, such as an anti-PD-Ll antibody or an antigen-binding fragment thereof, and an antisense compound targeted to STAT3 in a subject in need thereof.

SEQUENCE LISTING

<110>  AstraZeneca AB

<120>  COMBINATION

<130>  200252-EP-ETD

<160>  38

<170>  PatentIn version 3.5

<210>  1
<211>  4978
<212>  DNA
<213>  Homo sapiens

<400>  1
ggtttccgga gctgcggcgg cgcagactgg gagggggagc cgggggttcc gacgtcgcag      60

ccgagggaac aagccccaac cggatcctgg acaggcaccc cggcttggcg ctgtctctcc     120

ccctcggctc ggagaggccc ttcggcctga gggagcctcg ccgcccgtcc ccggcacacg     180

cgcagccccg gcctctcggc ctctgccgga gaaacagttg ggacccctga ttttagcagg     240

atggcccaat ggaatcagct acagcagctt gacacacggt acctggagca gctccatcag     300

ctctacagtg acagcttccc aatggagctg cggcagtttc tggccccttg gattgagagt     360

caagattggg catatgcggc cagcaaagaa tcacatgcca ctttggtgtt tcataatctc     420

ctgggagaga ttgaccagca gtatagccgc ttcctgcaag agtcgaatgt tctctatcag     480

cacaatctac gaagaatcaa gcagtttctt cagagcaggt atcttgagaa gccaatggag     540

attgcccgga ttgtggcccg gtgcctgtgg gaagaatcac gccttctaca gactgcagcc     600

actgcggccc agcaaggggg ccaggccaac caccccacag cagccgtggt gacggagaag     660

cagcagatgc tggagcagca ccttcaggat gtccggaaga gagtgcagga tctagaacag     720

aaaatgaaag tggtagagaa tctccaggat gactttgatt tcaactataa aaccctcaag     780

agtcaaggag acatgcaaga tctgaatgga aacaaccagt cagtgaccag gcagaagatg     840

cagcagctgg aacagatgct cactgcgctg gaccagatgc ggagaagcat cgtgagtgag     900

ctggcggggc ttttgtcagc gatggagtac gtgcagaaaa ctctcacgga cgaggagctg     960

gctgactgga agaggcggca acagattgcc tgcattggag gcccgcccaa catctgccta    1020

gatcggctag aaaactggat aacgtcatta gcagaatctc aacttcagac ccgtcaacaa    1080

attaagaaac tggaggagtt gcagcaaaaa gtttcctaca aaggggaccc cattgtacag    1140

caccggccga tgctggagga gagaatcgtg gagctgttta gaaacttaat gaaaagtgcc    1200

tttgtggtgg agcggcagcc ctgcatgccc atgcatcctg accggcccct cgtcatcaag    1260

accggcgtcc agttcactac taaagtcagg ttgctggtca aattccctga gttgaattat    1320

cagcttaaaa ttaaagtgtg cattgacaaa gactctgggg acgttgcagc tctcagagga 1380

tcccggaaat ttaacattct gggcacaaac acaaaagtga tgaacatgga agaatccaac 1440

aacggcagcc tctctgcaga attcaaacac ttgaccctga gggagcagag atgtgggaat 1500

gggggccgag ccaattgtga tgcttccctg attgtgactg aggagctgca cctgatcacc 1560

tttgagaccg aggtgtatca ccaaggcctc aagattgacc tagagaccca ctccttgcca 1620

gttgtggtga tctccaacat ctgtcagatg ccaaatgcct gggcgtccat cctgtggtac 1680

aacatgctga ccaacaatcc caagaatgta aactttttta ccaagccccc aattggaacc 1740

tgggatcaag tggccgaggt cctgagctgg cagttctcct ccaccaccaa gcgaggactg 1800

agcatcgagc agctgactac actggcagag aaactcttgg gacctggtgt gaattattca 1860

gggtgtcaga tcacatgggc taaattttgc aaagaaaaca tggctggcaa gggcttctcc 1920

ttctgggtct ggctggacaa tatcattgac cttgtgaaaa agtacatcct ggccctttgg 1980

aacgaagggt acatcatggg ctttatcagt aaggagcggg agcgggccat cttgagcact 2040

aagcctccag gcaccttcct gctaagattc agtgaaagca gcaaagaagg aggcgtcact 2100

ttcacttggg tggagaagga catcagcggt aagacccaga tccagtccgt ggaaccatac 2160

acaaagcagc agctgaacaa catgtcattt gctgaaatca tcatgggcta taagatcatg 2220

gatgctacca atatcctggt gtctccactg gtctatctct atcctgacat tcccaaggag 2280

gaggcattcg gaaagtattg tcggccagag agccaggagc atcctgaagc tgacccaggt 2340

agcgctgccc catacctgaa gaccaagttt atctgtgtga caccaacgac ctgcagcaat 2400

accattgacc tgccgatgtc cccccgcact ttagattcat tgatgcagtt tggaaataat 2460

ggtgaaggtg ctgaaccctc agcaggaggg cagtttgagt ccctcacctt tgacatggag 2520

ttgacctcgg agtgcgctac ctcccccatg tgaggagctg agaacggaag ctgcagaaag 2580

atacgactga ggcgcctacc tgcattctgc cacccctcac acagccaaac cccagatcat 2640

ctgaaactac taactttgtg gttccagatt ttttttaatc tcctacttct gctatctttg 2700

agcaatctgg gcacttttaa aaatagagaa atgagtgaat gtgggtgatc tgcttttatc 2760

taaatgcaaa taaggatgtg ttctctgaga cccatgatca ggggatgtgg cggggggtgg 2820

ctagagggag aaaaaggaaa tgtcttgtgt tgttttgttc ccctgccctc ctttctcagc 2880

agctttttgt tattgttgtt gttgttctta gacaagtgcc tcctggtgcc tgcggcatcc 2940

ttctgcctgt ttctgtaagc aaatgccaca ggccacctat agctacatac tcctggcatt 3000

gcacttttta accttgctga catccaaata gaagatagga ctatctaagc cctaggtttc 3060

tttttaaatt aagaaataat aacaattaaa gggcaaaaaa cactgtatca gcatagcctt 3120

tctgtattta agaaacttaa gcagccgggc atggtggctc acgcctgtaa tcccagcact 3180

ttgggaggcc gaggcggatc ataaggtcag gagatcaaga ccatcctggc taacacggtg 3240

aaaccccgtc tctactaaaa gtacaaaaaa ttagctgggt gtggtggtgg gcgcctgtag 3300

tcccagctac tcgggaggct gaggcaggag aatcgcttga acctgagagg cggaggttgc 3360

agtgagccaa aattgcacca ctgcacactg cactccatcc tgggcgacag tctgagactc 3420

tgtctcaaaa aaaaaaaaaa aaaaaagaaa cttcagttaa cagcctcctt ggtgctttaa 3480

gcattcagct tccttcaggc tggtaattta tataatccct gaaacgggct tcaggtcaaa 3540

cccttaagac atctgaagct gcaacctggc ctttggtgtt gaaataggaa ggtttaagga 3600

gaatctaagc attttagact tttttttata aatagactta ttttcctttg taatgtattg 3660

gccttttagt gagtaaggct gggcagaggg tgcttacaac cttgactccc tttctccctg 3720

gacttgatct gctgtttcag aggctaggtt gtttctgtgg gtgccttatc agggctggga 3780

tacttctgat tctggcttcc ttcctgcccc accctcccga ccccagtccc cctgatcctg 3840

ctagaggcat gtctccttgc gtgtctaaag gtccctcatc ctgtttgttt taggaatcct 3900

ggtctcagga cctcatggaa gaagaggggg agagagttac aggttggaca tgatgcacac 3960

tatggggccc cagcgacgtg tctggttgag ctcagggaat atggttctta gccagtttct 4020

tggtgatatc cagtggcact tgtaatggcg tcttcattca gttcatgcag ggcaaaggct 4080

tactgataaa cttgagtctg ccctcgtatg agggtgtata cctggcctcc ctctgaggct 4140

ggtgactcct ccctgctggg gccccacagg tgaggcagaa cagctagagg gcctccccgc 4200

ctgcccgcct tggctggcta gctcgcctct cctgtgcgta tgggaacacc tagcacgtgc 4260

tggatgggct gcctctgact cagaggcatg gccggatttg gcaactcaaa accaccttgc 4320

ctcagctgat cagagtttct gtggaattct gtttgttaaa tcaaattagc tggtctctga 4380

attaaggggg agacgacctt ctctaagatg aacagggttc gccccagtcc tcctgcctgg 4440

agacagttga tgtgtcatgc agagctctta cttctccagc aacactcttc agtacataat 4500

aagcttaact gataaacaga atatttagaa aggtgagact tgggcttacc attgggttta 4560

aatcataggg acctagggcg agggttcagg gcttctctgg agcagatatt gtcaagttca 4620

tggccttagg tagcatgtat ctggtcttaa ctctgattgt agcaaaagtt ctgagaggag 4680

ctgagccctg ttgtggccca ttaaagaaca gggtcctcag gccctgcccg cttcctgtcc 4740

actgccccct ccccatcccc agcccagccg agggaatccc gtgggttgct tacctaccta 4800

taaggtggtt tataagctgc tgtcctggcc actgcattca aattccaatg tgtacttcat 4860

agtgtaaaaa tttatattat tgtgaggttt tttgtctttt tttttttttt tttttttggg 4920

tatattgctg tatctacttt aacttccaga aataaacgtt atataggaac cgtaaaaa 4978

<210> 2
<211> 16
<212> DNA

<213>  Artificial Sequence

<220>
<223>  Synthetic oligonucleotide (AZD9150)

<400>  2
ctatttggat gtcagc                                                       16

<210>  3
<211>  108
<212>  PRT
<213>  Homo sapiens
<223>  MEDI4736 VL; Sequence 77 from PCT/US2010/058007

<400>  3

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Arg Val Ser Ser Ser
            20                  25                  30

Tyr Leu Ala Trp Tyr Gln Gln Lys Pro Gly Gln Ala Pro Arg Leu Leu
        35                  40                  45

Ile Tyr Asp Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Leu Pro
                85                  90                  95

Trp Thr Phe Gly Gln Gly Thr Lys Val Glu Ile Lys
            100                 105

<210>  4
<211>  121
<212>  PRT
<213>  Homo sapiens
<223>  MEDI4736 VH; Sequence 72 from PCT/US2010/058007

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val

        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser
        115                 120

<210> 5
<211> 5
<212> PRT
<213> Homo sapiens
<223> MEDI4736 VH CDR1; Sequence 73 from PCT/US2010/058007

<400> 5

Arg Tyr Trp Met Ser
1               5

<210> 6
<211> 17
<212> PRT
<213> Homo sapiens
<223> MEDI4736 VH CDR2;Sequence 74 from PCT/US2010/058007

<400> 6

Asn Ile Lys Gln Asp Gly Ser Glu Lys Tyr Tyr Val Asp Ser Val Lys
1               5                   10                  15

Gly

<210> 7
<211> 12
<212> PRT
<213> Homo sapiens
<223> MEDI4736 VH CDR3;Sequence 75 from PCT/US2010/058007

<400> 7

Glu Gly Gly Trp Phe Gly Glu Leu Ala Phe Asp Tyr
1               5                   10

<210>  8
<211>  12
<212>  PRT
<213>  Homo sapiens
<223>  MEDI4736 VL CDR1;Sequence 78 from PCT/US2010/058007

<400>  8

Arg Ala Ser Gln Arg Val Ser Ser Ser Tyr Leu Ala
1               5                   10

<210>  9
<211>  7
<212>  PRT
<213>  Homo sapiens
<223>  MEDI4736 VL CDR2;Sequence 79 from PCT/US2010/058007

<400>  9

Asp Ala Ser Ser Arg Ala Thr
1               5

<210>  10
<211>  9
<212>  PRT
<213>  Homo sapiens
<223>  MEDI4736 VL CDR3;Sequence 80 from PCT/US2010/058007

<400>  10

Gln Gln Tyr Gly Ser Leu Pro Trp Thr
1               5

<210>  11
<211>  16
<212>  DNA
<213>  Artificial sequence
<223>  Synthetic oligonucleotide (murine STAT3 ASO)

<400>  11
gaaattcatt cttcca                                                       16

<210>  12
<211>  16
<212>  DNA
<213>  Artificial sequence
<223>  Synthetic oligonucleotide (murine control ASO)

<400>  12
ggctactacg ccgtca                                                       16

<210>  13
<211>  139
<212>  PRT
<213>  Homo sapiens
<223>  Tremelimumab VL

<400>  13

Pro Ser Ser Leu Ser Ala Ser Val Gly Asp Arg Val Thr Ile Thr Cys
1               5                   10                  15

Arg Ala Ser Gln Ser Ile Asn Ser Tyr Leu Asp Trp Tyr Gln Gln Lys
            20                  25                  30

Pro Gly Lys Ala Pro Lys Leu Leu Ile Tyr Ala Ala Ser Ser Leu Gln
        35                  40                  45

Ser Gly Val Pro Ser Arg Phe Ser Gly Ser Gly Ser Gly Thr Asp Phe
    50                  55                  60

Thr Leu Thr Ile Ser Ser Leu Gln Pro Glu Asp Phe Ala Thr Tyr Tyr
65                  70                  75                  80

Cys Gln Gln Tyr Tyr Ser Thr Pro Phe Thr Phe Gly Pro Gly Thr Lys
                85                  90                  95

Val Glu Ile Lys Arg Thr Val Ala Ala Pro Ser Val Phe Ile Phe Pro
            100                 105                 110

Pro Ser Asp Glu Gln Leu Lys Ser Gly Thr Ala Ser Val Val Cys Leu
        115                 120                 125

Leu Asn Asn Phe Tyr Pro Arg Glu Ala Lys Val
    130                 135

<210>  14
<211>  167
<212>  PRT
<213>  Homo sapiens
<223>  Tremelimumab VH

<400>  14

Gly Val Val Gln Pro Gly Arg Ser Leu Arg Leu Ser Cys Ala Ala Ser
1               5                   10                  15

Gly Phe Thr Phe Ser Ser Tyr Gly Met His Trp Val Arg Gln Ala Pro
            20                  25                  30

Gly Lys Gly Leu Glu Trp Val Ala Val Ile Trp Tyr Asp Gly Ser Asn
        35                  40                  45

Lys Tyr Tyr Ala Asp Ser Val Lys Gly Arg Phe Thr Ile Ser Arg Asp
    50                  55                  60

Asn Ser Lys Asn Thr Leu Tyr Leu Gln Met Asn Ser Leu Arg Ala Glu

65                  70                  75                  80

Asp Thr Ala Val Tyr Tyr Cys Ala Arg Asp Pro Arg Gly Ala Thr Leu
                85                  90                  95

Tyr Tyr Tyr Tyr Tyr Gly Met Asp Val Trp Gly Gln Gly Thr Thr Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His
                165

<210>  15
<211>  10
<212>  PRT
<213>  Homo sapiens
<223>  Tremelimumab VH CDR1

<400>  15

Gly Phe Thr Phe Ser Ser Tyr Gly Met His
1               5                   10

<210>  16
<211>  15
<212>  PRT
<213>  Homo sapiens
<223>  Tremelimumab VH CDR2

<400>  16

Val Ile Trp Tyr Asp Gly Ser Asn Lys Tyr Tyr Ala Asp Ser Val
1               5                   10                  15

<210>  17
<211>  16
<212>  PRT
<213>  Homo sapiens
<223>  Tremelimumab VH CDR3

<400>  17

Asp Pro Arg Gly Ala Thr Leu Tyr Tyr Tyr Tyr Tyr Gly Met Asp Val
1               5                   10                  15

<210>  18
<211>  11
<212>  PRT
<213>  Homo sapiens
<223>  Tremelimumab VL CDR1

<400>  18

Arg Ala Ser Gln Ser Ile Asn Ser Tyr Leu Asp
1               5                   10


<210>  19
<211>  7
<212>  PRT
<213>  Homo sapiens
<223>  Tremelimumab VL CDR2

<400>  19

Ala Ala Ser Ser Leu Gln Ser
1               5


<210>  20
<211>  9
<212>  PRT
<213>  Homo sapiens
<223>  Tremelimumab VL CDR3

<400>  20

Gln Gln Tyr Tyr Ser Thr Pro Phe Thr
1               5


<210>  21
<211>  223
<212>  PRT
<213>  Homo sapiens
<223>  CTLA-4 polypeptide - GenBank Accession No. AAL07473.1

<400>  21

<210>  22
<211>  277
<212>  PRT
<213>  Homo sapiens
<223>  OX40 polypeptide

<400>  22

Met Cys Val Gly Ala Arg Arg Leu Gly Arg Gly Pro Cys Ala Ala Leu
1               5                   10                  15

Leu Leu Leu Gly Leu Gly Leu Ser Thr Val Thr Gly Leu His Cys Val
            20                  25                  30

Gly Asp Thr Tyr Pro Ser Asn Asp Arg Cys Cys His Glu Cys Arg Pro
        35                  40                  45

Gly Asn Gly Met Val Ser Arg Cys Ser Arg Ser Gln Asn Thr Val Cys
    50                  55                  60

Arg Pro Cys Gly Pro Gly Phe Tyr Asn Asp Val Val Ser Ser Lys Pro

65                  70                  75                  80

Cys Lys Pro Cys Thr Trp Cys Asn Leu Arg Ser Gly Ser Glu Arg Lys
                85                  90                  95

Gln Leu Cys Thr Ala Thr Gln Asp Thr Val Cys Arg Cys Arg Ala Gly
            100                 105                 110

Thr Gln Pro Leu Asp Ser Tyr Lys Pro Gly Val Asp Cys Ala Pro Cys
        115                 120                 125

Pro Pro Gly His Phe Ser Pro Gly Asp Asn Gln Ala Cys Lys Pro Trp
    130                 135                 140

Thr Asn Cys Thr Leu Ala Gly Lys His Thr Leu Gln Pro Ala Ser Asn
145                 150                 155                 160

Ser Ser Asp Ala Ile Cys Glu Asp Arg Asp Pro Pro Ala Thr Gln Pro
                165                 170                 175

Gln Glu Thr Gln Gly Pro Pro Ala Arg Pro Ile Thr Val Gln Pro Thr
            180                 185                 190

Glu Ala Trp Pro Arg Thr Ser Gln Gly Pro Ser Thr Arg Pro Val Glu
        195                 200                 205

Val Pro Gly Gly Arg Ala Val Ala Ala Ile Leu Gly Leu Gly Leu Val
    210                 215                 220

Leu Gly Leu Leu Gly Pro Leu Ala Ile Leu Leu Ala Leu Tyr Leu Leu
225                 230                 235                 240

Arg Arg Asp Gln Arg Leu Pro Pro Asp Ala His Lys Pro Pro Gly Gly
                245                 250                 255

Gly Ser Phe Arg Thr Pro Ile Gln Glu Glu Gln Ala Asp Ala His Ser
            260                 265                 270

Thr Leu Ala Lys Ile
        275

<210>  23
<211>  183
<212>  PRT
<213>  Homo sapiens
<223>  OX40 ligand

<400>  23

Met Glu Arg Val Gln Pro Leu Glu Glu Asn Val Gly Asn Ala Ala Arg
1               5                   10                  15

Pro Arg Phe Glu Arg Asn Lys Leu Leu Leu Val Ala Ser Val Ile Gln
            20                  25                  30

Gly Leu Gly Leu Leu Leu Cys Phe Thr Tyr Ile Cys Leu His Phe Ser
        35                  40                  45

Ala Leu Gln Val Ser His Arg Tyr Pro Arg Ile Gln Ser Ile Lys Val
    50                  55                  60

Gln Phe Thr Glu Tyr Lys Lys Glu Lys Gly Phe Ile Leu Thr Ser Gln
65                  70                  75                  80

Lys Glu Asp Glu Ile Met Lys Val Gln Asn Asn Ser Val Ile Ile Asn
                85                  90                  95

Cys Asp Gly Phe Tyr Leu Ile Ser Leu Lys Gly Tyr Phe Ser Gln Glu
            100                 105                 110

Val Asn Ile Ser Leu His Tyr Gln Lys Asp Glu Glu Pro Leu Phe Gln
        115                 120                 125

Leu Lys Lys Val Arg Ser Val Asn Ser Leu Met Val Ala Ser Leu Thr
    130                 135                 140

Tyr Lys Asp Lys Val Tyr Leu Asn Val Thr Thr Asp Asn Thr Ser Leu
145                 150                 155                 160

Asp Asp Phe His Val Asn Gly Gly Glu Leu Ile Leu Ile His Gln Asn
                165                 170                 175

Pro Gly Glu Phe Cys Val Leu
            180

<210>  24
<211>  410
<212>  PRT
<213>  Artificial sequence
<223>  OX40 ligand protein; corresponds to SEQ ID NO: 8 in U.S. Patent
       7,959,925

<400>  24

<210>  25
<211>  107
<212>  PRT
<213>  Homo sapiens
<223>  humanised anti-OX40 antibody VL
<400>  25

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Lys Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Ser Ala Leu Pro Trp

85                  90                  95

Thr Phe Gly Gln Gly Thr Lys Val Glu Ile Lys
            100                 105

<210>  26
<211>  107
<212>  PRT
<213>  Homo sapiens
<223>  humanised anti-OX40 antibody VL
<400>  26

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Asn Trp Tyr Gln Gln Lys Pro Gly Lys Ala Val Lys Leu Leu Ile
        35                  40                  45

Tyr Tyr Thr Ser Lys Leu His Ser Gly Val Pro Ser Arg Phe Ser Gly
    50                  55                  60

Ser Gly Ser Arg Thr Asp Tyr Thr Leu Thr Ile Ser Ser Leu Gln Pro
65                  70                  75                  80

Glu Asp Phe Ala Thr Tyr Tyr Cys Gln Gln Gly Ser Ala Leu Pro Trp
                85                  90                  95

Thr Phe Gly Gln Gly Thr Lys Val Glu Ile Lys
            100                 105

<210>  27
<211>  121
<212>  PRT
<213>  Homo sapiens
<223>  humanised anti-OX40 antibody VH
<400>  27

Gln Val Gln Leu Gln Glu Ser Gly Pro Gly Leu Val Lys Pro Ser Gln
1               5                   10                  15

Thr Leu Ser Leu Thr Cys Ala Val Tyr Gly Gly Ser Phe Ser Ser Gly
            20                  25                  30

Tyr Trp Asn Trp Ile Arg Lys His Pro Gly Lys Gly Leu Glu Tyr Ile
        35                  40                  45

Gly Tyr Ile Ser Tyr Asn Gly Ile Thr Tyr His Asn Pro Ser Leu Lys
    50                  55                  60

Ser Arg Ile Thr Ile Asn Arg Asp Thr Ser Lys Asn Gln Tyr Ser Leu
65                  70                  75                  80

Gln Leu Asn Ser Val Thr Pro Glu Asp Thr Ala Val Tyr Tyr Cys Ala
                85                  90                  95

Arg Tyr Lys Tyr Asp Tyr Asp Gly Gly His Ala Met Asp Tyr Trp Gly
            100                 105                 110

Gln Gly Thr Leu Val Thr Val Ser Ser
        115                 120

<210>  28
<211>  451
<212>  PRT
<213>  Homo sapiens
<223>  humanised anti-Ox40 antibody heavy chain

<400>  28

Gln Val Gln Leu Gln Glu Ser Gly Pro Gly Leu Val Lys Pro Ser Gln
1               5                   10                  15

Thr Leu Ser Leu Thr Cys Ala Val Tyr Gly Gly Ser Phe Ser Ser Gly
            20                  25                  30

Tyr Trp Asn Trp Ile Arg Lys His Pro Gly Lys Gly Leu Glu Tyr Ile
        35                  40                  45

Gly Tyr Ile Ser Tyr Asn Gly Ile Thr Tyr His Asn Pro Ser Leu Lys
    50                  55                  60

Ser Arg Ile Thr Ile Asn Arg Asp Thr Ser Lys Asn Gln Tyr Ser Leu
65                  70                  75                  80

Gln Leu Asn Ser Val Thr Pro Glu Asp Thr Ala Val Tyr Tyr Cys Ala
                85                  90                  95

Arg Tyr Lys Tyr Asp Tyr Asp Gly Gly His Ala Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val

145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  29
<211>  214
<212>  PRT
<213>  Homo sapiens
<223>  humanised anti-OX40 antibody Light chain

<400>  29

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Lys Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Ser Ala Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  30
<211>  412
<212>  PRT
<213>  Homo sapiens
<223>  humanised anti-OX40 antibody

<400>  30

Ala Pro Leu Ala Thr Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys Glu Leu Leu Gly Gly Gly Ser Ile
225                 230                 235                 240

Lys Gln Ile Glu Asp Lys Ile Glu Glu Ile Leu Ser Lys Ile Tyr His
                245                 250                 255

Ile Glu Asn Glu Ile Ala Arg Ile Lys Lys Leu Ile Gly Glu Arg Gly
            260                 265                 270

His Gly Gly Gly Ser Asn Ser Gln Val Ser His Arg Tyr Pro Arg Phe
        275                 280                 285

Gln Ser Ile Lys Val Gln Phe Thr Glu Tyr Lys Lys Glu Lys Gly Phe
    290                 295                 300

Ile Leu Thr Ser Gln Lys Glu Asp Glu Ile Met Lys Val Gln Asn Asn
305                 310                 315                 320

Ser Val Ile Ile Asn Cys Asp Gly Phe Tyr Leu Ile Ser Leu Lys Gly
                325                 330                 335

Tyr Phe Ser Gln Glu Val Asn Ile Ser Leu His Tyr Gln Lys Asp Glu
            340                 345                 350

Glu Pro Leu Phe Gln Leu Lys Lys Val Arg Ser Val Asn Ser Leu Met
        355                 360                 365

Val Ala Ser Leu Thr Tyr Lys Asp Lys Val Tyr Leu Asn Val Thr Thr
    370                 375                 380

Asp Asn Thr Ser Leu Asp Asp Phe His Val Asn Gly Gly Glu Leu Ile
385                 390                 395                 400

Leu Ile His Gln Asn Pro Gly Glu Phe Cys Val Leu

405                 410

<210>  31
<211>  1206
<212>  DNA
<213>  Homo sapiens
<223>  DNA Sequence of huIgG4FcPTF2OX40L (5\222 to 3\222 Open Reading Frame)

<400>  31
gagagcaagt acggccctcc ctgcccccct tgccctgccc ccgagttcct gggcggacct      60

agcgtgttcc tgttcccccc caagcccaag gacaccctga tgatcagcag aacccccgag     120

gtgacctgcg tggtggtgga cgtgtcccag gaggaccccg aggtccagtt taattggtac     180

gtggacggcg tggaagtgca taacgccaag accaagccca gagaggagca gttcaacagc     240

acctacagag tggtgtccgt gctgaccgtg ctgcaccagg actggctgaa cggcaaggaa     300

tacaagtgca aggtctccaa caagggcctg cctagcagca tcgagaagac catcagcaag     360

gccaagggcc agccacggga gccccaggtc tacaccctgc cacctagcca agaggagatg     420

accaagaacc aggtgtccct gacctgtctg gtgaaaggct tctatcccag cgatatcgcc     480

gtggagtggg agagcaacgg ccagcccgag aacaactaca agaccacccc ccctgtgctg     540

gacagcgacg gcagcttctt cctgtactcc agactgaccg tggacaagtc cagatggcag     600

gagggcaacg tcttcagctg ctccgtgatg cacgaggccc tgcacaacca ctacacccag     660

aagtccctga gcctgagcct gggcaaggac caggataaga tcgaggctct gtcctccaag     720

gtgcagcagc tggaacggtc catcggcctg aaggacctgg ccatggctga cctggaacag     780

aaagtgctgg aaatggaagc ctccacacag gtgtcacaca gatacccccg gatccagtcc     840

attaaggtgc agttcaccga gtacaagaaa gagaagggct ttatcctgac ctcccagaaa     900

gaggacgaga tcatgaaggt gcagaacaac tccgtgatca tcaactgcga cgggttctac     960

ctgatctccc tgaagggcta cttcagccag gaagtgaaca tctccctgca ctaccagaag    1020

gacgaggaac ccctgttcca gctgaagaaa gtgcggagcg tgaactccct gatggtggcc    1080

tctctgacct acaaggacaa ggtgtacctg aacgtgacca ccgacaacac ctccctggac    1140

gacttccacg tgaacggcgg cgagctgatc ctgatccacc agaaccctgg cgagttctgc    1200

gtgctg                                                               1206

<210>  32
<211>  402
<212>  PRT
<213>  Homo Sapiens
<223>  Amino Acid Sequence of huIgG4FcPTF2OX40L (N to C terminus)

<400>  32

Glu Ser Lys Tyr Gly Pro Pro Cys Pro Pro Cys Pro Ala Pro Glu Phe

1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Asp Gln Asp Lys Ile Glu Ala Leu Ser Ser Lys
225                 230                 235                 240

Val Gln Gln Leu Glu Arg Ser Ile Gly Leu Lys Asp Leu Ala Met Ala
                245                 250                 255

Asp Leu Glu Gln Lys Val Leu Glu Met Glu Ala Ser Thr Gln Val Ser
            260                 265                 270

His Arg Tyr Pro Arg Ile Gln Ser Ile Lys Val Gln Phe Thr Glu Tyr
        275                 280                 285

Lys Lys Glu Lys Gly Phe Ile Leu Thr Ser Gln Lys Glu Asp Glu Ile
    290                 295                 300

Met Lys Val Gln Asn Asn Ser Val Ile Ile Asn Cys Asp Gly Phe Tyr
305                 310                 315                 320

Leu Ile Ser Leu Lys Gly Tyr Phe Ser Gln Glu Val Asn Ile Ser Leu
                325                 330                 335

His Tyr Gln Lys Asp Glu Glu Pro Leu Phe Gln Leu Lys Lys Val Arg
            340                 345                 350

Ser Val Asn Ser Leu Met Val Ala Ser Leu Thr Tyr Lys Asp Lys Val
        355                 360                 365

Tyr Leu Asn Val Thr Thr Asp Asn Thr Ser Leu Asp Asp Phe His Val
    370                 375                 380

Asn Gly Gly Glu Leu Ile Leu Ile His Gln Asn Pro Gly Glu Phe Cys
385                 390                 395                 400

Val Leu

<210>  33
<211>  1206
<212>  DNA
<213>  Homo sapiens
<223>  DNA Sequence of huIgG4FcPTF2OX40L F180A (5\222 to 3\222 Open Reading
Frame)

<400>  33
gagagcaagt acggccctcc ctgcccccct tgccctgccc ccgagttcct gggcggacct      60

agcgtgttcc tgttcccccc caagcccaag gacaccctga tgatcagcag aacccccgag      120

gtgacctgcg tggtggtgga cgtgtcccag gaggaccccg aggtccagtt taattggtac      180

gtggacggcg tggaagtgca taacgccaag accaagccca gagaggagca gttcaacagc      240

acctacagag tggtgtccgt gctgaccgtg ctgcaccagg actggctgaa cggcaaggaa      300

tacaagtgca aggtctccaa caagggcctg cctagcagca tcgagaagac catcagcaag      360

gccaagggcc agccacggga gccccaggtc tacaccctgc cacctagcca agaggagatg      420

accaagaacc aggtgtccct gacctgtctg gtgaaaggct tctatcccag cgatatcgcc      480

gtggagtggg agagcaacgg ccagcccgag aacaactaca agaccacccc ccctgtgctg 540

gacagcgacg gcagcttctt cctgtactcc agactgaccg tggacaagtc cagatggcag 600

gagggcaacg tcttcagctg ctccgtgatg cacgaggccc tgcacaacca ctacacccag 660

aagtccctga gcctgagcct gggcaaggac caggataaga tcgaggctct gtcctccaag 720

gtgcagcagc tggaacggtc catcggcctg aaggacctgg ccatggctga cctggaacag 780

aaagtgctgg aaatggaagc ctccacacag gtgtcacaca gatacccccg gatccagtcc 840

attaaggtgc agttcaccga gtacaagaaa gagaagggct ttatcctgac ctcccagaaa 900

gaggacgaga tcatgaaggt gcagaacaac tccgtgatca tcaactgcga cgggttctac 960

ctgatctccc tgaagggcta cttcagccag gaagtgaaca tctccctgca ctaccagaag 1020

gacgaggaac ccctgttcca gctgaagaaa gtgcggagcg tgaactccct gatggtggcc 1080

tctctgacct acaaggacaa ggtgtacctg aacgtgacca ccgacaacac ctccctggac 1140

gacttccacg tgaacggcgg cgagctgatc ctgatccacc agaaccctgg cgaggcctgc 1200

gtgctg 1206

<210> 34
<211> 1206
<212> PRT
<213> Homo Sapiens
<223> Amino Acid Sequence of huIgG4PFcTF2OX40L F180A (N to C terminus)

<400> 34

Gly Ala Gly Ala Gly Cys Ala Ala Gly Thr Ala Cys Gly Gly Cys Cys
1               5                   10                  15

Cys Thr Cys Cys Cys Thr Gly Cys Cys Cys Cys Cys Cys Thr Thr Gly
            20                  25                  30

Cys Cys Cys Thr Gly Cys Cys Cys Cys Cys Gly Ala Gly Thr Thr Cys
        35                  40                  45

Cys Thr Gly Gly Gly Cys Gly Gly Ala Cys Cys Thr Ala Gly Cys Gly
    50                  55                  60

Thr Gly Thr Thr Cys Cys Thr Gly Thr Thr Cys Cys Cys Cys Cys Cys
65                  70                  75                  80

Cys Ala Ala Gly Cys Cys Cys Ala Ala Gly Gly Ala Cys Ala Cys Cys
                85                  90                  95

Cys Thr Gly Ala Thr Gly Ala Thr Cys Ala Gly Cys Ala Gly Ala Ala
            100                 105                 110

Cys Cys Cys Cys Cys Gly Ala Gly Gly Thr Gly Ala Cys Cys Thr Gly
        115                 120                 125

Cys Gly Thr Gly Gly Thr Gly Gly Thr Gly Gly Ala Cys Gly Thr Gly
    130                 135                 140

Thr Cys Cys Cys Ala Gly Gly Ala Gly Gly Ala Cys Cys Cys Cys Gly
145                 150                 155                 160

Ala Gly Gly Thr Cys Cys Ala Gly Thr Thr Thr Ala Ala Thr Thr Gly
                165                 170                 175

Gly Thr Ala Cys Gly Thr Gly Gly Ala Cys Gly Gly Cys Gly Thr Gly
            180                 185                 190

Gly Ala Ala Gly Thr Gly Cys Ala Thr Ala Ala Cys Gly Cys Cys Ala
        195                 200                 205

Ala Gly Ala Cys Cys Ala Ala Gly Cys Cys Cys Ala Gly Ala Gly Ala
    210                 215                 220

Gly Gly Ala Gly Cys Ala Gly Thr Thr Cys Ala Ala Cys Ala Gly Cys
225                 230                 235                 240

Ala Cys Cys Thr Ala Cys Ala Gly Ala Gly Thr Gly Gly Thr Gly Thr
                245                 250                 255

Cys Cys Gly Thr Gly Cys Thr Gly Ala Cys Cys Gly Thr Gly Cys Thr
            260                 265                 270

Gly Cys Ala Cys Cys Ala Gly Gly Ala Cys Thr Gly Gly Cys Thr Gly
        275                 280                 285

Ala Ala Cys Gly Gly Cys Ala Ala Gly Gly Ala Ala Thr Ala Cys Ala
    290                 295                 300

Ala Gly Thr Gly Cys Ala Ala Gly Gly Thr Cys Thr Cys Cys Ala Ala
305                 310                 315                 320

Cys Ala Ala Gly Gly Gly Cys Cys Thr Gly Cys Cys Thr Ala Gly Cys
                325                 330                 335

Ala Gly Cys Ala Thr Cys Gly Ala Gly Ala Ala Gly Ala Cys Cys Ala
            340                 345                 350

Thr Cys Ala Gly Cys Ala Ala Gly Gly Cys Cys Ala Ala Gly Gly Gly
        355                 360                 365

Cys Cys Ala Gly Cys Cys Ala Cys Gly Gly Gly Ala Gly Cys Cys Cys
    370                 375                 380

Cys Ala Gly Gly Thr Cys Thr Ala Cys Ala Cys Cys Cys Thr Gly Cys
385                 390                 395                 400

Cys Ala Cys Cys Thr Ala Gly Cys Cys Ala Ala Gly Ala Gly Gly Ala
                405                 410                 415

Gly Ala Thr Gly Ala Cys Cys Ala Ala Gly Ala Ala Cys Cys Ala Gly
            420                 425                 430

Gly Thr Gly Thr Cys Cys Cys Thr Gly Ala Cys Cys Thr Gly Thr Cys
        435                 440                 445

Thr Gly Gly Thr Gly Ala Ala Ala Gly Gly Cys Thr Thr Cys Thr Ala
    450                 455                 460

Thr Cys Cys Cys Ala Gly Cys Gly Ala Thr Ala Thr Cys Gly Cys Cys
465                 470                 475                 480

Gly Thr Gly Gly Ala Gly Thr Gly Gly Gly Ala Gly Ala Gly Cys Ala
                485                 490                 495

Ala Cys Gly Gly Cys Cys Ala Gly Cys Cys Cys Gly Ala Gly Ala Ala
            500                 505                 510

Cys Ala Ala Cys Thr Ala Cys Ala Ala Gly Ala Cys Cys Ala Cys Cys
        515                 520                 525

Cys Cys Cys Cys Cys Thr Gly Thr Gly Cys Thr Gly Gly Ala Cys Ala
    530                 535                 540

Gly Cys Gly Ala Cys Gly Gly Cys Ala Gly Cys Thr Thr Cys Thr Thr
545                 550                 555                 560

Cys Cys Thr Gly Thr Ala Cys Thr Cys Cys Ala Gly Ala Cys Thr Gly
                565                 570                 575

Ala Cys Cys Gly Thr Gly Gly Ala Cys Ala Ala Gly Thr Cys Cys Ala
            580                 585                 590

Gly Ala Thr Gly Gly Cys Ala Gly Gly Ala Gly Gly Gly Cys Ala Ala
        595                 600                 605

Cys Gly Thr Cys Thr Thr Cys Ala Gly Cys Thr Gly Cys Thr Cys Cys

610                 615                 620

Gly Thr Gly Ala Thr Gly Cys Ala Cys Gly Ala Gly Gly Cys Cys Cys
625                 630                 635                 640

Thr Gly Cys Ala Cys Ala Ala Cys Cys Ala Cys Thr Ala Cys Ala Cys
                645                 650                 655

Cys Cys Ala Gly Ala Ala Gly Thr Cys Cys Cys Thr Gly Ala Gly Cys
            660                 665                 670

Cys Thr Gly Ala Gly Cys Cys Thr Gly Gly Gly Cys Ala Ala Gly Gly
        675                 680                 685

Ala Cys Cys Ala Gly Gly Ala Thr Ala Ala Gly Ala Thr Cys Gly Ala
    690                 695                 700

Gly Gly Cys Thr Cys Thr Gly Thr Cys Cys Thr Cys Cys Ala Ala Gly
705                 710                 715                 720

Gly Thr Gly Cys Ala Gly Cys Ala Gly Cys Thr Gly Gly Ala Ala Cys
                725                 730                 735

Gly Gly Thr Cys Cys Ala Thr Cys Gly Gly Cys Cys Thr Gly Ala Ala
            740                 745                 750

Gly Gly Ala Cys Cys Thr Gly Gly Cys Cys Ala Thr Gly Gly Cys Thr
        755                 760                 765

Gly Ala Cys Cys Thr Gly Gly Ala Ala Cys Ala Gly Ala Ala Ala Gly
    770                 775                 780

Thr Gly Cys Thr Gly Gly Ala Ala Ala Thr Gly Gly Ala Ala Gly Cys
785                 790                 795                 800

Cys Thr Cys Cys Ala Cys Ala Cys Ala Gly Gly Thr Gly Thr Cys Ala
                805                 810                 815

Cys Ala Cys Ala Gly Ala Thr Ala Cys Cys Cys Cys Cys Gly Gly Ala
            820                 825                 830

Thr Cys Cys Ala Gly Thr Cys Cys Ala Thr Thr Ala Ala Gly Gly Thr
        835                 840                 845

Gly Cys Ala Gly Thr Thr Cys Ala Cys Cys Gly Ala Gly Thr Ala Cys
    850                 855                 860

Ala Ala Gly Ala Ala Ala Gly Ala Gly Ala Ala Gly Gly Gly Cys Thr
865                 870                 875                 880

Thr Thr Ala Thr Cys Cys Thr Gly Ala Cys Cys Thr Cys Cys Cys Ala
                885                 890                 895

Gly Ala Ala Ala Gly Ala Gly Gly Ala Cys Gly Ala Gly Ala Thr Cys
            900                 905                 910

Ala Thr Gly Ala Ala Gly Gly Thr Gly Cys Ala Gly Ala Ala Cys Ala
        915                 920                 925

Ala Cys Thr Cys Cys Gly Thr Gly Ala Thr Cys Ala Thr Cys Ala Ala
    930                 935                 940

Cys Thr Gly Cys Gly Ala Cys Gly Gly Gly Thr Thr Cys Thr Ala Cys
945                 950                 955                 960

Cys Thr Gly Ala Thr Cys Thr Cys Cys Cys Thr Gly Ala Ala Gly Gly
                965                 970                 975

Gly Cys Thr Ala Cys Thr Thr Cys Ala Gly Cys Cys Ala Gly Gly Ala
            980                 985                 990

Ala Gly Thr Gly Ala Ala Cys Ala  Thr Cys Thr Cys Cys  Cys Thr Gly
        995                 1000                1005

Cys Ala  Cys Thr Ala Cys Cys  Ala Gly Ala Ala Gly  Gly Ala Cys
    1010                1015                1020

Gly Ala  Gly Gly Ala Ala Cys  Cys Cys Cys Thr Gly  Thr Thr Cys
    1025                1030                1035

Cys Ala  Gly Cys Thr Gly Ala  Ala Gly Ala Ala Ala  Gly Thr Gly
    1040                1045                1050

Cys Gly  Gly Ala Gly Cys Gly  Thr Gly Ala Ala Cys  Thr Cys Cys
    1055                1060                1065

Cys Thr  Gly Ala Thr Gly Gly  Thr Gly Gly Cys Cys  Thr Cys Thr
    1070                1075                1080

Cys Thr  Gly Ala Cys Cys Thr  Ala Cys Ala Ala Gly  Gly Ala Cys
    1085                1090                1095

Ala Ala  Gly Gly Thr Gly Thr  Ala Cys Cys Thr Gly  Ala Ala Cys
    1100                1105                1110

Gly Thr Gly Ala Cys Cys Ala Cys Cys Gly Ala Cys Ala Ala Cys
    1115                1120                1125

Ala Cys Cys Thr Cys Cys Cys Thr Gly Gly Ala Cys Gly Ala Cys
    1130                1135                1140

Thr Thr Cys Cys Ala Cys Gly Thr Gly Ala Ala Cys Gly Gly Cys
    1145                1150                1155

Gly Gly Cys Gly Ala Gly Cys Thr Gly Ala Thr Cys Cys Thr Gly
    1160                1165                1170

Ala Thr Cys Cys Ala Cys Cys Ala Gly Ala Ala Cys Cys Cys Thr
    1175                1180                1185

Gly Gly Cys Gly Ala Gly Gly Cys Cys Thr Gly Cys Gly Thr Gly
    1190                1195                1200

Cys Thr Gly
    1205

<210> 35
<211> 1200
<212> DNA
<213> Homo sapiens
<223> DNA Sequence of huIgG1TF2OX40L (5\222 to 3\222 Open Reading Frame)

<400> 35
gataagaccc acacctgtcc cccttgtcct gcccctgaac tgctgggcgg accttccgtg 60

ttcctgttcc ccccaaagcc caaggacacc ctgatgatct cccggacccc cgaagtgacc 120

tgcgtggtgg tggatgtgtc ccacgaggac cctgaagtga agttcaattg gtacgtggac 180

ggcgtggaag tgcacaacgc caagaccaag cccagagagg aacagtacaa ctccacctac 240

cgggtggtgt ccgtgctgac cgtgctgcac caggattggc tgaacggcaa agagtacaag 300

tgcaaggtgt ccaacaaggc cctgcctgcc cccatcgaaa agaccatctc caaggccaag 360

ggccagcccc gggaacccca ggtgtacaca ctgcccccta gccgggaaga gatgaccaag 420

aaccaggtgt ccctgacctg tctcgtgaag ggcttctacc cctccgatat cgccgtggaa 480

tgggagtcca acggccagcc tgagaacaac tacaagacca ccccccctgt gctggactcc 540

gacggctcat tcttcctgta ctccaagctg acagtggaca agtcccggtg gcagcagggc 600

aacgtgttct cctgctccgt gatgcacgag gccctgcaca accactacac ccagaagtcc 660

ctgtccctga gccccggcaa ggaccaggat aagatcgagg ctctgtcctc caaggtgcag 720

cagctggaac ggtccatcgg cctgaaggac ctggccatgg ctgacctgga acagaaagtg 780

ctggaaatgg aagcctccac acaggtgtca cacagatacc cccggatcca gtccattaag 840

gtgcagttca ccgagtacaa gaaagagaag ggctttatcc tgacctccca gaaagaggac 900

gagatcatga aggtgcagaa caactccgtg atcatcaact gcgacgggtt ctacctgatc 960

tccctgaagg gctacttcag ccaggaagtg aacatctccc tgcactacca gaaggacgag 1020

gaacccctgt tccagctgaa gaaagtgcgg agcgtgaact ccctgatggt ggcctctctg 1080

acctacaagg acaaggtgta cctgaacgtg accaccgaca acacctccct ggacgacttc 1140

cacgtgaacg gcggcgagct gatcctgatc caccagaacc ctggcgagtt ctgcgtgctg 1200

<210> 36
<211> 400
<212> PRT
<213> Homo sapiens
<223> Amino Acid Sequence of huIgG1FcTF2OX40L (N to C terminus)

<400> 36

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Asp Gln Asp Lys Ile Glu Ala Leu Ser Ser Lys Val Gln
225                 230                 235                 240

Gln Leu Glu Arg Ser Ile Gly Leu Lys Asp Leu Ala Met Ala Asp Leu
                245                 250                 255

Glu Gln Lys Val Leu Glu Met Glu Ala Ser Thr Gln Val Ser His Arg
            260                 265                 270

Tyr Pro Arg Ile Gln Ser Ile Lys Val Gln Phe Thr Glu Tyr Lys Lys
        275                 280                 285

Glu Lys Gly Phe Ile Leu Thr Ser Gln Lys Glu Asp Glu Ile Met Lys
    290                 295                 300

Val Gln Asn Asn Ser Val Ile Ile Asn Cys Asp Gly Phe Tyr Leu Ile
305                 310                 315                 320

Ser Leu Lys Gly Tyr Phe Ser Gln Glu Val Asn Ile Ser Leu His Tyr
                325                 330                 335

Gln Lys Asp Glu Glu Pro Leu Phe Gln Leu Lys Lys Val Arg Ser Val
            340                 345                 350

Asn Ser Leu Met Val Ala Ser Leu Thr Tyr Lys Asp Lys Val Tyr Leu
        355                 360                 365

Asn Val Thr Thr Asp Asn Thr Ser Leu Asp Asp Phe His Val Asn Gly
    370                 375                 380

Gly Glu Leu Ile Leu Ile His Gln Asn Pro Gly Glu Phe Cys Val Leu
385                 390                 395                 400

<210>  37
<211>  1248

<212>  DNA
<213>  Mus musculis
<223>  DNA Sequence of mouse construct mIgG1mTF2mOX40L (5\222 to 3\222 Open
Reading Frame)

<400>  37
gtgcctagag attgcggctg caagccctgc atctgcaccg tgcccgaggt gtccagcgtg      60

ttcatcttcc cacccaagcc caaggacgtg ctgaccatca ccctgacccc caaagtgacc     120

tgcgtggtgg tggacatcag caaggacgac cccgaggtgc agttcagttg gttcgtggac     180

gacgtggaag tgcacaccgc ccagacccag cccagagagg aacagttcaa cagcaccttc     240

agaagcgtgt ccgagctgcc catcatgcac caggactggc tgaacggcaa agaattcaag     300

tgcagagtga acagcgccgc cttccctgcc cccatcgaga aaaccatcag caagaccaag     360

ggcagaccca aggcccccca ggtgtacacc atccccccac ccaaagaaca gatggccaag     420

gacaaggtgt ccctgacctg catgatcacc gatttcttcc cagaggacat caccgtggaa     480

tggcagtgga acggccagcc cgccgagaac tacaagaaca cccagcccat catggacacc     540

gacggcagct acttcgtgta cagcaagctg aacgtgcaga agtccaactg ggaggccggc     600

aacaccttca cctgtagcgt gctgcacgag ggcctgcaca accaccacac cgagaagtcc     660

ctgagccaca gccccggcaa gcggctggac caggacaaga tcgaggccct gagcaacaag     720

gtgcagcagc tggaacggtc tatcggcctg aaggacctgg ctatggccga cctggaacag     780

aaagtgtctg agctggaagt gtccaccagc agccccgcca aggaccctcc catccagaga     840

ctgagaggcg ccgtgaccag atgcgaggac ggccagctgt tcatcagcag ctacaagaac     900

gagtaccaga ccatggaagt gcagaacaac agcgtggtca tcaagtgcga cggcctgtac     960

atcatctacc tcaagggcag cttcttccag gaagtgaaga tcgacctgca cttcagagag    1020

gaccacaacc ccatcagcat ccccatgctg aacgacggca gacggatcgt gttcaccgtg    1080

gtggctagcc tggccttcaa ggacaaagtg tatctgaccg tgaacgcccc cgacaccctg    1140

tgcgagcatc tgcagatcaa cgacggcgag ctgatcgtgg tgcagctgac ccccggctac    1200

tgtgcccctg agggcagcta ccacagcacc gtgaaccagg tgcccctg                 1248


<210>  38
<211>  416
<212>  PRT
<213>  Mus musculis
<223>  Amino Acid Sequence of mouse construct mIgG1FcmTF2mOX40L (N to C
terminus)

<400>  38

Val Pro Arg Asp Cys Gly Cys Lys Pro Cys Ile Cys Thr Val Pro Glu
1               5                   10                  15


Val Ser Ser Val Phe Ile Phe Pro Pro Lys Pro Lys Asp Val Leu Thr

20                  25                  30

Ile Thr Leu Thr Pro Lys Val Thr Cys Val Val Val Asp Ile Ser Lys
        35                  40                  45

Asp Asp Pro Glu Val Gln Phe Ser Trp Phe Val Asp Asp Val Glu Val
    50                  55                  60

His Thr Ala Gln Thr Gln Pro Arg Glu Glu Gln Phe Asn Ser Thr Phe
65                  70                  75                  80

Arg Ser Val Ser Glu Leu Pro Ile Met His Gln Asp Trp Leu Asn Gly
                85                  90                  95

Lys Glu Phe Lys Cys Arg Val Asn Ser Ala Ala Phe Pro Ala Pro Ile
            100                 105                 110

Glu Lys Thr Ile Ser Lys Thr Lys Gly Arg Pro Lys Ala Pro Gln Val
        115                 120                 125

Tyr Thr Ile Pro Pro Pro Lys Glu Gln Met Ala Lys Asp Lys Val Ser
    130                 135                 140

Leu Thr Cys Met Ile Thr Asp Phe Phe Pro Glu Asp Ile Thr Val Glu
145                 150                 155                 160

Trp Gln Trp Asn Gly Gln Pro Ala Glu Asn Tyr Lys Asn Thr Gln Pro
                165                 170                 175

Ile Met Asp Thr Asp Gly Ser Tyr Phe Val Tyr Ser Lys Leu Asn Val
            180                 185                 190

Gln Lys Ser Asn Trp Glu Ala Gly Asn Thr Phe Thr Cys Ser Val Leu
        195                 200                 205

His Glu Gly Leu His Asn His His Thr Glu Lys Ser Leu Ser His Ser
    210                 215                 220

Pro Gly Lys Arg Leu Asp Gln Asp Lys Ile Glu Ala Leu Ser Asn Lys
225                 230                 235                 240

Val Gln Gln Leu Glu Arg Ser Ile Gly Leu Lys Asp Leu Ala Met Ala
                245                 250                 255

Asp Leu Glu Gln Lys Val Ser Glu Leu Glu Val Ser Thr Ser Ser Pro
            260                 265                 270

Ala Lys Asp Pro Pro Ile Gln Arg Leu Arg Gly Ala Val Thr Arg Cys
        275                 280                 285

Glu Asp Gly Gln Leu Phe Ile Ser Ser Tyr Lys Asn Glu Tyr Gln Thr
    290                 295                 300

Met Glu Val Gln Asn Asn Ser Val Val Ile Lys Cys Asp Gly Leu Tyr
305                 310                 315                 320

Ile Ile Tyr Leu Lys Gly Ser Phe Phe Gln Glu Val Lys Ile Asp Leu
                325                 330                 335

His Phe Arg Glu Asp His Asn Pro Ile Ser Ile Pro Met Leu Asn Asp
            340                 345                 350

Gly Arg Arg Ile Val Phe Thr Val Val Ala Ser Leu Ala Phe Lys Asp
        355                 360                 365

Lys Val Tyr Leu Thr Val Asn Ala Pro Asp Thr Leu Cys Glu His Leu
    370                 375                 380

Gln Ile Asn Asp Gly Glu Leu Ile Val Val Gln Leu Thr Pro Gly Tyr
385                 390                 395                 400

Cys Ala Pro Glu Gly Ser Tyr His Ser Thr Val Asn Gln Val Pro Leu
                405                 410                 415

## Description

### Sequence Listing

The present application is being filed along with a Sequence Listing in electronic format. The Sequence Listing is provided as a file entitled 200252_ST25PCT.txt created 19 Oct 2015, which is 65 kb in size. The information in the electronic format of the sequence listing is incorporated herein by reference in its entirety.

### Field

In certain embodiments, methods, compounds, and compositions for treating cancer, for example B-cell lymphoma, in an animal, by administering an agent capable of inhibiting expression of STAT3 mRNA or protein and an immunomudulatory agent, are provided herein. In particular embodiments, the agent capable of inhibiting expression of STAT3 mRNA or protein is selective for inhibiting expression of STAT3 mRNA or protein. In particular embodiments, the combination therapy involves administration to a patient in need thereof an antisense compound targeted to STAT3 and an agent (such as an antibody) capable of inhibiting binding of PD-L1 ligand to its receptor. Such methods, compounds, and compositions are useful to treat, prevent, or ameliorate B-cell lymphoma and other cancers that are susceptible to response with an immune checkpoint inhibitor.

### BACKGROUND OF THE INVENTION

The role of the immune system, in particular T cell-mediated cytotoxicity, in tumor control is well recognized. There is mounting evidence that T cells control tumor growth and survival in cancer patients, both in early and late stages of the disease. However, tumor-specific T-cell responses are difficult to mount and sustain in cancer patients.

T cell pathways receiving significant attention to date signal through cytotoxic T lymphocyte antigen-4 (CTLA-4, CD152), programmed death ligand 1 (PD-L1, also known as B7-H1 or CD274), and OX40 (CD134; TNFRSF4).

CTLA-4 is expressed on activated T cells and serves as a co-inhibitor to keep T cell responses in check following CD28-mediated T cell activation. CTLA-4 is believed to regulate the amplitude of the early activation of naive and memory T cells following TCR engagement and to be part of a central inhibitory pathway that affects both antitumor immunity and autoimmunity. CTLA-4 is expressed exclusively on T cells, and the expression of its ligands CD80 (B7.1) and CD86 (B7.2), is largely restricted to antigen-presenting cells, T cells, and other immune mediating cells. CTLA-4 belongs to a class of molecules known as immune checkpoint proteins. Antagonistic anti-CTLA-4 antibodies that block the CTLA-4 signaling pathway have been reported to enhance T cell activation. One such antibody, ipilimumab, was approved by the FDA in 2011 for the treatment of metastatic melanoma. Another anti-CTLA-4 antibody, tremelimumab, was tested in phase III trials for the treatment of advanced melanoma, but did not significantly increase the overall survival of patients compared to the standard of care (temozolomide or dacarbazine) at that time.

PD-L1 and PD1 belong to a class of molecules known as immune checkpoint proteins. These proteins are cell surface-bound ligand-receptor pairs that, in healthy individuals, dampen immune responses to prevent an over-reaction of the immune system. Cancer cells often hijack the normal PD-L1-PD1 immune checkpoint mechanism by overexpressing the ligand PD-L1, which binds to PD1 on effector CD8 T cells, thereby preventing the T cells from mounting an immune response to the tumor. PD-L1 is expressed in a broad range of cancers with a high frequency. Tumor PD-L1 over expression correlates with poor prognosis in a number of cancers (see e.g. Hamid and Carvajal. Expert Opin. Biol. Ther. 13(6):847-861, 2013).

Antibodies that block the interaction between PD-L1 and its receptors are able to relieve PD-L1 -dependent immunosuppressive effects and enhance the cytotoxic activity of antitumor T cells *in vitro.* MEDI4736 (also known as durvalumab) is a human monoclonal antibody directed against human PD-L1 that is capable of blocking the binding of PD-L1 to both the PD-1 and CD80 receptors.

Anti-PD-Ll and anti-PDl therapeutic blocking antibodies are being trialled and have shown clinical benefit in a number of tumor types including lung cancer, melanoma, renal cell carcinoma, bladder cancer, gastric cancer, head and neck cancer, etc; but only a minority of patients respond to these therapies (e.g. see, Brahmer et al., New Engl. J. Med. 366(26):2455-2465, 2012; Harvey. Clinical Pharmacology & Therapeutics 96(2): 214-223, 2014). This lack of response has been attributed to other modes of immunosuppression that override PD1/PD-L1 effects and combination approaches that involve immunosuppression are being considered (Dolan et al., Cancer Control 21(3)231-237).

OX40 is a tumor necrosis factor receptor (TNFR) found primarily on activated CD4+ and CD8+ T cells, regulatory T cells (Treg), and natural killer (NK) cells. Signaling through OX40 on activated CD4+ and CD8+ T cells leads to enhanced cytokine production, granzyme and perforin release, and expansion of effector and memory T-cell pools. In addition, OX40 signaling on Treg cells inhibits expansion of Tregs, shuts down the induction of Tregs, and blocks Treg-suppressive function.

Despite the potential of immunomodulators or immune checkpoint inhibitors for the treatment of cancers, there is a need in the art for improving upon responses to these agents for treating cancers.

### SUMMARY OF THE INVENTION

As described below, the present invention features methods of treating cancer (e.g., lymphoma) with an immunomodulator, such as an immune checkpoint inhibitor, and an antisense compound targeted to STAT3 in a subject in need thereof. Particular immunomodulators include an anti-PD-Ll antibody (or an antigen-binding fragment thereof), an anti-PDl antibody (or an antigen-binding fragment thereof), an anti-CTLA-4 antibody (or an antigen-binding fragment thereof) and an OX40 agonist ((*e.g.*, an OX40 ligand fusion protein, or an OX40 agonist antibody or antigen-binding fragment thereof). The subject can be any mammal. In one embodiment the subject is a human. Also provided are combination products and kits, each comprising an immunomodulator and an antisense compound targeted to STAT3, for use in treating one or more types of cancer. In particular embodiments the antisense compound targeted to STAT3 is a single-stranded antisense oligonucleotide (ASO).

Antibodies that selectively bind CTLA-4, PD-L1 or PD-1, or inhibit the binding or activation of CTLA-4, PD-L1 or PD-1 are useful in the methods of the invention. Antibodies that selectively bind and activate OX40 are useful in the methods of the invention.

Anti-PD-Ll antibodies are known in the art. Exemplary anti-PD-Ll antibodies include: MEDI4736 (durvalumab), MPDL3280A, BMS936559, 2.7A4, AMP-714 and MDX-1105.

Anti-PD-1 antibodies are known in the art. Exemplary anti-PD-1 antibodies include: nivolumab, pembrolizumab, pidilizumab and MPDL3280A.

Anti-CTLA-4 antibodies are known in the art. Exemplary anti-CTLA-4 antibodies include: tremelimumab and ipilimumab, also termed MDX-010 (or BMS-734016).

OX-40 agonists are known in the art. Exemplary OX-40 agonists include: OX40L FP.

AZD9150 is an antisense oligonucleotide and an example of an antisense compound targeted to STAT3.

In one embodiment the combination involves the antisense oligonucleotide AZD9150 and at least one immunomodulator selected from the group consisting of: MED14736, MPDL3280A, BMS936559, 2.7A4, AMP-714, MDX-1105, nivolumab, pembrolizumab, pidilizumab, MPDL3280A, tremelimumab, ipilimumab and OX40L FP.

In one embodiment the combination involves the anti-PD-Ll antibody MEDI4736 and the antisense oligonucleotide AZD9150.

In one embodiment the combination involves the antisense oligonucleotide AZD9150, the anti-PD-Ll antibody MEDI4736 (durvalumab) and the anti-CTLA-4 antibody tremelimumab.

PDL-1 has been implicated in helping a variety of cancers evade immune surveillance by the body. As such, the present invention is predicted to be of benefit in treating any cancer.

Examples of types of cancer that the combination treatment is proposed for include: lung cancer, including non small cell lung cancer (NSCLC), breast cancer, including triple negative, ovarian cancer, including serous, pancreatic cancer, colorectal cancer, hepatocellular carcinoma (HCC), head and neck cancer, including head and neck squamous cell carcinoma (HNSCC), and lymphoma, including diffuse large B-cell carcinoma (DLBCL).

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). Standard techniques may be used for chemical synthesis, and chemical analysis. Where permitted, all patents, applications, published applications and other publications, GENBANK Accession Numbers and associated sequence information obtainable through databases such as National Center for Biotechnology Information (NCBI) and other data referred to throughout in the disclosure herein are incorporated by reference for the portions of the document discussed herein, as well as in their entirety.

Unless otherwise indicated, the following terms have the following meanings:
"2'-deoxynucleoside" means a nucleoside comprising 2'-H furanosyl sugar moiety, as found naturally occurring in deoxyribonucleosides (DNA). In certain embodiments, a 2'-deoxynucleoside may comprise a modified nucleobase or may comprise an RNA nucleobase (e.g., uracil).

"2'-substituted nucleoside" means a nucleoside comprising a substituent at the 2'-position other than H or OH. Unless otherwise indicated, a 2'-substituted nucleoside is not a bicyclic nucleoside.

"5'-methylcytosine" means a cytosine modified with a methyl group attached to the 5' position. A 5-methylcytosine is a modified nucleobase.

As used herein, the term "About" is understood as within a range of normal tolerance in the art, and generally means within ±10%, such as within 9%, 8%, 7%, 6%, 5%, 4%, 3%, 2%, 1%, 0.5%, 0.1%, 0.05%, or 0.01% of the stated value. For example, if it is stated, "the compounds affected at least about 70% inhibition of STAT3", it is implied that the STAT3 levels are inhibited within a range of 63% and 77%. If it is stated that the compound is used at about 20mg/kg, it covers the range 18-22mg/kg inclusive. Unless otherwise clear from context, all numerical values provided herein are modified by the term about.

"Administered concomitantly" refers to the co-administration of two agents in any manner in which the pharmacological effects of both are manifest in the patient at the same time. Concomitant administration does not require that both agents be administered in a single pharmaceutical composition, in the same dosage form, or by the same route of administration. The effects of both agents need not manifest themselves at the same time. The effects need only be overlapping for a period of time and need not be coextensive.

"Agent" refers to a substance, such as a compound, antisense oligonucleotide or antibody (and the like) that is capable of producting an effect.

"Animal" refers to a human or non-human animal, including, but not limited to, mice, rats, rabbits, dogs, cats, pigs, and non-human primates, including, but not limited to, monkeys and chimpanzees.

The term "antibody," as used in this disclosure, refers to an immunoglobulin or a fragment or a derivative thereof, and encompasses any polypeptide comprising an antigen-binding site, regardless whether it is produced *in vitro* or *in vivo.* The term includes, but is not limited to, polyclonal, monoclonal, monospecific, polyspecific, non-specific, humanized, single-chain, chimeric, synthetic, recombinant, hybrid, mutated, and grafted antibodies. Unless otherwise modified by the term "intact," as in "intact antibodies," for the purposes of this disclosure, the term "antibody" also includes antibody fragments such as Fab, F(ab')₂, Fv, scFv, Fd, dAb, and other antibody fragments that retain antigen-binding function, *i.e.,* the ability to bind, for example, CTLA-4, PD-L1 specifically. Typically, such fragments would comprise an antigen-binding domain.

By "anti-CTLA-4 antibody" is meant an antibody that selectively binds a CTLA-4 polypeptide. Exemplary anti- CTLA-4 antibodies are described for example at US Patent Nos. 6,682,736; 7,109,003; 7,123,281; 7,411,057; 7,824,679; 8,143,379; 7,807,797; and 8,491,895 (Tremelimumab is 11.2.1, therein), which are herein incorporated by reference. Tremelimumab (U.S. Patent No. 6,682,736) is an exemplary anti-CTLA-4 antibody.

### Tremelimumab sequences:

VL - SEQ ID NO: 13.
VH - SEQ ID NO: 14.
VH CDR1 - SEQ ID NO: 15; VH CDR2 - SEQ ID NO: 16; VH CDR3 - SEQ ID NO: 17.
VL CDR1 - SEQ ID NO: 18; VL CDR2 - SEQ ID NO: 19; VL CDR3 - SEQ ID NO: 20.

By "anti-PD-L1 antibody" is meant an antibody that selectively binds a PD-L1 polypeptide. Exemplary anti-PD-Ll antibodies are described for example at WO 2011/066389, US20130034559 / US8779108 and US20140356353which are herein incorporated by reference. MEDI4736 is an exemplary anti-PD-Ll antibody. The sequences are provided in the sequence listing below (e.g., SEQ ID NOs. 3-10). Other anti-PD-Ll antibodies include BMS-936559 (Bristol-Myers Squibb) and MPDL3280A (Roche).

The terms "antigen-binding domain," "antigen-binding fragment," and "binding fragment" refer to a part of an antibody molecule that comprises amino acids responsible for the specific binding between the antibody and the antigen. In instances, where an antigen is large, the antigen-binding domain may only bind to a part of the antigen. A portion of the antigen molecule that is responsible for specific interactions with the antigen-binding domain is referred to as "epitope" or "antigenic determinant." An antigen-binding domain typically comprises an antibody light chain variable region (V_{L}) and an antibody heavy chain variable region (V_{H}), however, it does not necessarily have to comprise both. For example, a so-called Fd antibody fragment consists only of a V_{H} domain, but still retains some antigen-binding function of the intact antibody.

Binding fragments of an antibody are produced by recombinant DNA techniques, or by enzymatic or chemical cleavage of intact antibodies. Binding fragments include Fab, Fab', F(ab')2, Fv, and single-chain antibodies. An antibody other than a "bispecific" or "bifunctional" antibody is understood to have each of its binding sites identical. Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a F(ab')2 fragment in which the two arms of the antibody molecule remain linked and comprise two-antigen binding sites. The F(ab')2 fragment has the ability to crosslink antigen. "Fv" when used herein refers to the minimum fragment of an antibody that retains both antigen-recognition and antigen-binding sites. "Fab" when used herein refers to a fragment of an antibody that comprises the constant domain of the light chain and the CHI domain of the heavy chain.

The term "mAb" refers to monoclonal antibody. Antibodies of the invention comprise without limitation whole native antibodies, bispecific antibodies; chimeric antibodies; Fab, Fab', single chain V region fragments (scFv), fusion polypeptides, and unconventional antibodies.

"Antisense compound" means an oligomeric compound that is is capable of undergoing hybridization to a target nucleic acid through hydrogen bonding. Examples of antisense compounds include single-stranded and double-stranded compounds, such as, antisense oligonucleotides, siRNAs, shRNAs, snoRNAs, miRNAs, meroduplex (mdRNA) and satellite repeats.

"An antisense compound targeted to STAT3" means an oligomeric compound that is is capable of undergoing hybridization to STAT3 target nucleic acid through hydrogen bonding

"Antisense oligonucleotide" means a single-stranded oligonucleotide having a nucleobase sequence that permits hybridization to a corresponding region or segment of a target nucleic acid.

"Anti-tumor activity" means any biological activity that reduces or stabilizes the proliferation or survival of a tumor cell. In one embodiment, the anti-tumor activity is an anti-tumor immune response.

"Bicyclic sugar" means a furosyl ring modified by the bridging of two atoms. A bicyclic sugar is a modified sugar.

By "cancer" is meant a disease or disorder characterized by excess proliferation or reduced apoptosis. Illustrative cancers for which the invention can be used include, but are not limited to leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease, DLBCL), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, glioblastoma multiforme, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma).

"Chimeric antisense compound" means an antisense compound that has at least two chemically distinct regions.

"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration. In one embodiment, the co-administration is carried out so as to result in exposure of the patient to both drugs at the same time based on the pharmacokinetics of the drugs.

"Constrained ethyl nucleoside" (also cEt nucleoside) means a nucleoside comprising a bicyclic sugar moiety comprising a 4'-CH(CH₃)-O-2' bridge.

"Contiguous nucleobases" means nucleobases immediately adjacent to each other.

By "Disease" is meant any condition or disorder that damages, interferes with or dysregulates the normal function of a cell, tissue, or organ. In a disease such as cancer (e.g., lung cancer) the normal function of a cell tissue or organ is subverted to enable immune evasion and/or escape.

By "CTLA-4 polypeptide" is meant a polypeptide having at least 85% amino acid sequence identity to GenBank Accession No. AAL07473.1 or a fragment thereof, having T cell inhibitory activity. The sequence of AAL07473.1 is disclosed in SEQ ID NO: 21, herein.

By "CTLA-4 nucleic acid molecule" is meant a polynucleotide encoding a CTLA-4 polypeptide. An exemplary CTLA-4 polynucleotide is provided at GenBank Accession No. AAL07473.

"Diluent" means an ingredient in a composition that lacks pharmacological activity, but is pharmaceutically necessary or desirable. For example, the diluent in an injected composition may be a liquid, e.g. saline solution.

"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.

"Effective amount" means the amount of active pharmaceutical agent sufficient to effectuate a desired physiological outcome in an individual in need of the agent. The effective amount may vary among individuals depending on the health and physical condition of the individual to be treated, the taxonomic group of the individuals to be treated, the formulation of the composition, assessment of the individual's medical condition, and other relevant factors.

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as the "gap" and the external regions may be referred to as the "wings."

"Hybridization" means the annealing of complementary nucleic acid molecules. In certain embodiments, complementary nucleic acid molecules include an antisense compound and a target nucleic acid.

"Immune checkpoint inhibitor" means an agent that inhibits the CTLA-4 or PD-1 pathways, particular checkpoint inhibitors include antibodies that inhibit PD-1, PD-L1 or CTLA-4.

"Immunomodulatory agent" means an agent that enhances an immune response (e.g., anti-tumor immune response). Exemplary immunomodulatory agents of the invention include antibodies, such as an anti-CTLA-4 antibody, an anti-PD-Ll antibody, an anti-PD-1 antibody and antigenic fragments of any of these, and OX-40 agonists, including proteins, such as OX40 ligand fusion protein, or fragments thereof. In one embodiment, the immunomodulatory agent is an immune checkpoint inhibitor.

"Inhibiting STAT3" means reducing expression of STAT3 mRNA and/or protein levels in the presence of a STAT3 antisense compound, including a STAT3 antisense oligonucleotide, as compared to expression of STAT3 mRNA and/or protein levels in the absence of a STAT3 antisense compound, such as an antisense oligonucleotide.

"Individual" means a human or non-human animal selected for treatment or therapy.

"Internucleoside linkage" refers to the chemical bond between nucleosides.

"Linked nucleosides" means adjacent nucleosides which are bonded together.

"Modified internucleoside linkage" refers to a substitution or any change from a naturally occurring internucleoside bond (i.e. a phosphodiester internucleoside bond).

"Modified nucleobase" refers to any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

"Modified nucleotide" means a nucleotide having, independently, a modified sugar moiety, modified internucleoside linkage, or modified nucleobase. A "modified nucleoside" means a nucleoside having, independently, a modified sugar moiety or modified nucleobase.

"Modified oligonucleotide" means an oligonucleotide comprising a modified internucleoside linkage, a modified sugar, and/or a modified nucleobase.

"Modified sugar" refers to a substitution or change from a natural sugar.

"Naturally occurring internucleoside linkage" means a 3' to 5' phosphodiester linkage.

"Nucleic acid" refers to molecules composed of monomeric nucleotides. A nucleic acid includes ribonucleic acids (RNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase sequence" means the order of contiguous nucleobases independent of any sugar, linkage, or nucleobase modification.

"Nucleoside" means a nucleobase linked to a sugar.

"Nucleotide" means a nucleoside having a phosphate group covalently linked to the sugar portion of the nucleoside.

"Oligomeric compound" or "oligomer" means a polymer of linked monomeric subunits which is capable of hybridizing to at least a region of a nucleic acid molecule.

"Oligonucleotide" means a polymer of linked nucleosides each of which can be modified or unmodified, independent one from another.

"Overall survival" means the length of time from the start of treatment for a disease, such as cancer, that patients diagnosed with the disease are still alive. The overall survival figure is typically determined as an average from an appropriately sized clinical trial.

"OX40 polypeptide" means a polypeptide or fragment thereof having at least about 85% amino acid identity to NCBI Accession No. NP_003318. OX40 is a member of the TNFR-superfamily of receptors that is expressed on the surface of antigen-activated mammalian CD4+ and CD8+ T lymphocytes. See, for example, Paterson et al., Mol Immunol 24, 1281-1290 (1987); Mallett et al., EMBO J 9, 1063-1068 (1990); and Calderhead et al., J Immunol 151, 5261-5271 (1993)). OX40 is also referred to as CD134, ACT-4, and ACT35. OX40 receptor sequences are known in the art and are provided, for example, at GenBank Accession Numbers: AAB33944 or CAE11757. An exemplary human OX40 amino acid sequence is disclosed in SEQ ID NO: 22, herein.

"OX40 ligand" means a polypeptide or fragment thereof having at least about 85% amino acid identity to NCBI Accession No. NP 003317 and that specifically binds the OX40 receptor. See, for example, Baum P.R., et al. EMBO J. 13:3992-4001(1994)). The term OX40L includes the entire OX40 ligand, soluble OX40 ligand, and fusion proteins comprising a functionally active portion of OX40 ligand covalently linked to a second moiety, *e.g*., a protein domain. Also included within the definition of OX40L are variants which vary in amino acid sequence from naturally occurring OX4L but which retain the ability to specifically bind to the OX40 receptor. Further included within the definition of OX40L are variants which enhance the biological activity of OX40. OX40 ligand sequences are known in the art and are provided, for example, at GenBank Accession Numbers: NP_003318.
An exemplary human OX40 ligand amino acid sequence is disclosed in SEQ ID NO: 23, herein.

"OX40 agonist" means an OX40 ligand that specifically interacts with and increases the biological activity of the OX40 receptor. Desirably, the biological activity is increased by at least about 10%, 20%, 30%, 50%, 70%, 80%, 90%, 95%, or even 100%. In certain aspects, OX40 agonists as disclosed herein include OX40 binding polypeptides, such as anti-OX40 antibodies (e.g., OX40 agonist antibodies), OX40 ligands, or fragments or derivatives of these molecules.

"OX40 antibody" means an antibody that specifically binds OX40. OX40 antibodies include monoclonal and polyclonal antibodies that are specific for OX40 and antigen-binding fragments thereof. In certain aspects, anti-OX40 antibodies as described herein are monoclonal antibodies (or antigen-binding fragments thereof), e.g., murine, humanized, or fully human monoclonal antibodies. In one particular embodiment, the OX40 antibody is an OX40 receptor agonist, such as the mouse anti-human OX40 monoclonal antibody (9B12) described by Weinberg et al., J Immunother 29, 575-585 (2006). In other embodiments, the antibody which specifically binds to OX40, or an antigen-binding fragment thereof, binds to the same OX40 epitope as mAb 9B12.

"OX40 ligand fusion protein" means a protein that specifically binds the OX40 receptor and increases an immune response. In one embodiment, binding of an OX40 ligand fusion protein to the OX40 receptor enhances a tumor antigen specific immune response by boosting T-cell recognition. Exemplary OX40 ligand fusion proteins are described in U.S. Patent 7,959,925, entitled, "Trimeric OX40 Immunoglobulin Fusion Protein and Methods of Use." See, for example, U.S. Patent 7,959,925, SEQ ID NO. 8. This sequence is reproduced herein as SEQ ID NO: 24. Other OX40 ligand fusion proteins are described, for example, in US Patent 6,312,700. In one embodiment, an OX40 ligand fusion protein enhances tumor-specific T-cell immunity. In particular embodiments, the OX40 ligand fusion protein has an amino acid sequence disclosed in SEQ ID NO: 32, 34 or 36 (such variants are termed herein as OX40L FP)
"Parenteral administration" means administration through injection (e.g., bolus injection) or infusion. Parenteral administration includes subcutaneous administration, intravenous administration, intramuscular administration, intraarterial administration, intraperitoneal administration, or intracranial administration, e.g., intrathecal or intracerebroventricular administration.

"PD-L1 polypeptide" means a polypeptide or fragment thereof having at least about 85% amino acid identity to NCBI Accession No. NP_001254635, and having PD-1 and CD80 binding activity.

"PD-L1 nucleic acid molecule" means a polynucleotide encoding a PD-L1 polypeptide. An exemplary PD-L1 nucleic acid molecule sequence is provided at NCBI Accession No. NM 001267706.

"PD-L1 positive" means, in the context of immunohistochemistry, cells in a cancer sample exhibit staining for PD-L1. The level of positivity that is biologically significant can vary, based on tumor type, and on the immune status of the tumor environment.

"Peptide" means a molecule formed by linking at least two amino acids by amide bonds. Peptide refers to polypeptides and proteins.

"Pharmaceutical composition" means a mixture of substances suitable for administering to an individual. For example, a pharmaceutical composition may comprise one or more active pharmaceutical agents and a sterile aqueous solution. In certain embodiments, a pharmaceutical composition shows activity in free uptake assay in certain cell lines.

"Phosphorothioate linkage" means a linkage between nucleosides where the phosphodiester bond is modified by replacing one of the non-bridging oxygen atoms with a sulfur atom. A phosphorothioate linkage (P=S) is a modified internucleoside linkage.

"Portion" means a defined number of contiguous (i.e., linked) nucleobases of a nucleic acid. In certain embodiments, a portion is a defined number of contiguous nucleobases of a target nucleic acid. In certain embodiments, a portion is a defined number of contiguous nucleobases of an antisense compound.

"Prevent" refers to delaying or forestalling the onset or development of a disease, disorder, or condition for a period of time from minutes to indefinitely. Prevent also means reducing risk of developing a disease, disorder, or condition.

"Progression free survival" means the length of time during and after the treatment of a disease, such as cancer, that a patient lives with the disease but it does not get worse. The progression free survival figure is typically determined as an average from an appropriately sized clinical trial.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

By "reference" is meant a standard of comparison.

By "responsive" in the context of therapy is meant susceptible to treatment.

"Signal Transducer and Activator of Transcription 3 nucleic acid" or "STAT3 nucleic acid" means any nucleic acid encoding STAT3. For example, in certain embodiments, a STAT3 nucleic acid includes a DNA sequence encoding STAT3, an RNA sequence transcribed from DNA encoding STAT3 (including genomic DNA comprising introns and exons), and an mRNA sequence encoding STAT3. "STAT3 mRNA" means an mRNA encoding a STAT3 protein.

"Single-stranded oligonucleotide" means an oligonucleotide which is not hybridized to a complementary strand.

By "specifically binds" is meant a compound (*e.g*., antibody) that recognizes and binds a molecule (*e.g*., polypeptide), but which does not substantially recognize and bind other molecules in a sample, for example, a biological sample. For example, two molecules that specifically bind form a complex that is relatively stable under physiologic conditions. Specific binding is characterized by a high affinity and a low to moderate capacity as distinguished from nonspecific binding which usually has a low affinity with a moderate to high capacity. Typically, binding is considered specific when the affinity constant K_{A} is higher than 10⁶ M⁻¹, or more preferably higher than 10⁸ M⁻¹ If necessary, non-specific binding can be reduced without substantially affecting specific binding by varying the binding conditions. The appropriate binding conditions such as concentration of antibodies, ionic strength of the solution, temperature, time allowed for binding, concentration of a blocking agent (*e.g.,* serum albumin, milk casein), *etc.,* may be optimized by a skilled artisan using routine techniques.

"Specifically hybridizable" refers to an antisense compound having a sufficient degree of complementarity (pairing between nucleobases) between an antisense oligonucleotide and a target nucleic acid to induce a desired effect, while exhibiting minimal or no effects on non-target nucleic acids under conditions in which specific binding is desired, i.e., under physiological conditions in the case of *in vivo* assays and therapeutic treatments.

By "Subject" is meant a mammal, including, but not limited to, a human or non-human mammal, such as a bovine, equine, canine, ovine, or feline.

"Targeting" or "targeted" means directed at. With respect to an antibody it refers to the ability to bind to the reference protein. With respect to an antisense compound it refers to they ability to specifically hybridize to a target nucleic acid and induce a desired effect.

"Target nucleic acid," "target RNA," "target mRNA," and "target RNA transcript" all refer to a nucleic acid capable of being targeted by antisense compounds.

"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.

"Therapeutically effective amount" means an amount of a pharmaceutical agent that provides a therapeutic benefit to an individual.

"Treat", "treating," "treatment," and the like, refers to administering a pharmaceutical composition to reduce or ameliorate a disease, disorder, or condition and/or any symptom associated therewith. It will be appreciated that, although not precluded, treating a disorder or condition does not require that the disorder, condition or symptoms associated therewith be completely eliminated.

"Unmodified nucleotide" means a nucleotide composed of naturally occuring nucleobases, sugar moieties, and internucleoside linkages. In certain embodiments, an unmodified nucleotide is an RNA nucleotide (i.e. β-D-ribonucleosides) or a DNA nucleotide (i.e. β-D-deoxyribonucleoside).

Unless specifically stated, or obvious from context, as used herein, the term "or" is understood to be inclusive. Unless specifically stated or obvious from context, as used herein, the terms "a", "an", and "the" are understood to be singular or plural.

In this disclosure, "comprises," "comprising," "containing" and "having" and the like can have the meaning ascribed to them in U.S. Patent law and can mean "includes," "including," and the like; "consisting essentially of' or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

As used herein, the terms "determining", "assessing", "assaying", "measuring" and "detecting" refer to both quantitative and qualitative determinations, and as such, the term "determining" is used interchangeably herein with "assaying," "measuring," and the like. Where a quantitative determination is intended, the phrase "determining an amount" of an analyte and the like is used. Where a qualitative and/or quantitative determination is intended, the phrase "determining a level" of an analyte or "detecting" an analyte is used.

The terms "isolated", "purified," or "biologically pure" refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of this invention is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

### DETAILED DESCRIPTION OF THE INVENTION

As described below, the present invention features methods of treating cancer (e.g., lung cancer, such as non-small cell lung cancer (NSCLC); breast cancer, including triple negative; ovarian cancer, including serous; pancreatic cancer; colorectal cancer; lymphoma, such as diffuse large B-cell lung cancer (DLBCL) or Hodgkin's lymphoma; or head and neck cancer, such as head and neck squamous cell carcinoma (HNSCC)) with an immunomodulatory agent, such as an anti-PD-L1 antibody like MEDI4736, and an antisense compound targeted to STAT3, such as AZD9150, in a subject in need thereof.

### Anti-PD-Ll Antibodies

Antibodies that specifically bind and inhibit PD-L1 are useful in the present invention.

MEDI4736 is an exemplary anti-PD-Ll antibody that is selective for a PD-L1 polypeptide and blocks the binding of PD-L1 to the PD-1 and CD80 receptors. MEDI4736 can relieve PD-L1 - mediated suppression of human T-cell activation *in vitro* and inhibits tumor growth in a xenograft model via a T-cell dependent mechanism.

Information regarding MEDI4736 (or fragments thereof) for use in the methods provided herein can be found in U.S. Patent No. 8,779,108, the disclosure of which is incorporated herein by reference in its entirety. The fragment crystallizable (Fc) domain of MEDI4736 contains a triple mutation in the constant domain of the IgG1 heavy chain that reduces binding to the complement component C1q and the Fcγ receptors responsible for mediating antibody-dependent cell-mediated cytotoxicity (ADCC).

MEDI4736 and antigen-binding fragments thereof for use in the methods provided herein comprises a heavy chain and a light chain or a heavy chain variable region and a light chain variable region. In a specific aspect, MEDI4736 or an antigen-binding fragment thereof for use in the methods provided herein comprises a light chain variable region comprising the amino acid sequence of SEQ ID NO:3 and a heavy chain variable region comprising the amino acid sequence of SEQ ID NO:4. In a specific aspect, MEDI4736 or an antigen-binding fragment thereof for use in the methods provided herein comprises a heavy chain variable region and a light chain variable region, wherein the heavy chain variable region comprises the Kabat-defined CDR1, CDR2, and CDR3 sequences of SEQ ID NOs: 5-7, and wherein the light chain variable region comprises the Kabat-defined CDR1, CDR2, and CDR3 sequences of SEQ ID NOs: 8-10. Those of ordinary skill in the art would easily be able to identify Chothia-defined, Abm-defined or other CDR definitions known to those of ordinary skill in the art. In a specific aspect, MEDI4736 or an antigen-binding fragment thereof for use in the methods provided herein comprises the variable heavy chain and variable light chain CDR sequences of the 2.14H9OPT antibody as disclosed in WO 2011/066389 A1, which is herein incorporated by reference in its entirety.

There are numerous anti-PDL-1 antibodies in the published literature that could feature in the present invention, including compounds in development and/or in clinical trials such as: MEDI4736, MPDL3280A, BMS936559, 2.7A4, AMP-714 and MDX-1105. Patent specifications disclosing anti-PDL-1 antibodies that may be useful in the present invention include: WO2007/005874 (BMS/Medarex), WO01/14556 (Dana Farber), US2011/0271358 (Dana Farber), WO2010/036959 (Dana Farber), WO2010/077634 (Genentech), including issued U.S. Patent No. 8,217,149, US2012/0039906 (INSERM), WO2012/145493 (Amplimmune), U.S. Patent No. 8,779,108 (MedImmune - for MEDI4726 and 2.7A4), US20140044738 (Ampimmune - for AMP-714) and WO2009/089149 (John's Hopkins University). Each of these disclosures is herein incorporated by reference in its entirety.

### Anti-CTLA-4 Antibodies

Antibodies that specifically bind CTLA-4 and inhibit CTLA-4 activity are useful for enhancing an anti-tumor immune response. Information regarding tremelimumab (or antigen-binding fragments thereof) for use in the methods provided herein can be found in US 6,682,736 (where it is referred to as 11.2.1), the disclosure of which is incorporated herein by reference in its entirety. Tremelimumab (also known as CP-675,206, CP-675, CP-675206, and ticilimumab) is a human IgG₂ monoclonal antibody that is highly selective for CTLA-4 and blocks binding of CTLA-4 to CD80 (B7.1) and CD86 (B7.2). It has been shown to result in immune activation *in vitro* and some patients treated with tremelimumab have shown tumor regression.

Tremelimumab for use in the methods provided herein comprises a heavy chain and a light chain or a heavy chain variable region and a light chain variable region. In a specific aspect, tremelimumab or an antigen-binding fragment thereof for use in the methods provided herein comprises a light chain variable region comprising the amino acid sequences shown herein above and a heavy chain variable region comprising the amino acid sequence shown herein above. In a specific aspect, tremelimumab or an antigen-binding fragment thereof for use in the methods provided herein comprises a heavy chain variable region and a light chain variable region, wherein the heavy chain variable region comprises the Kabat-defined CDR1, CDR2, and CDR3 sequences shown herein above, and wherein the light chain variable region comprises the Kabat-defined CDR1, CDR2, and CDR3 sequences shown herein above. Those of ordinary skill in the art would easily be able to identify Chothia-defined, Abm-defined or other CDR definitions known to those of ordinary skill in the art. In a specific aspect, tremelimumab or an antigen-binding fragment thereof for use in the methods provided herein comprises the variable heavy chain and variable light chain CDR sequences of the 11.2.1 antibody as disclosed in US 6,682,736, which is herein incorporated by reference in its entirety.

Other anti-CTLA-4 antibodies are described, for example, in US 20070243184. In one embodiment, the anti-CTLA-4 antibody is Ipilimumab, also termed MDX-010; BMS-734016. **OX40 Agonists**
OX40 agonists interact with the OX40 receptor on CD4⁺ T-cells during, or shortly after, priming by an antigen resulting in an increased response of the CD4⁺ T-cells to the antigen. An OX40 agonist interacting with the OX40 receptor on antigen specific CD4⁺ T-cells can increase T cell proliferation as compared to the response to antigen alone. The elevated response to the antigen can be maintained for a period of time substantially longer than in the absence of an OX40 agonist. Thus, stimulation via an OX40 agonist enhances the antigen specific immune response by boosting T-cell recognition of antigens, *e.g*., tumor cells. OX40 agonists are described, for example, in U.S. Patent Nos. 6,312,700, 7,504,101, 7,622,444, and 7,959,925, which are incorporated herein by reference in their entireties. Methods of using such agonists in cancer treatment are described, for example, in WO/2013/119202 and in WO/2013/130102, each of which are incorporated herein by reference in its entirety.

OX40 agonists include, but are not limited to OX40 binding molecules, *e.g*., binding polypeptides, *e.g*., OX40 ligand ("OX40L") or an OX40-binding fragment, variant, or derivative thereof, such as soluble extracellular ligand domains and OX40L fusion proteins, and anti-OX40 antibodies (for example, monoclonal antibodies such as humanized monoclonal antibodies), or an antigen-binding fragment, variant or derivative thereof. Examples of anti-OX40 monoclonal antibodies are described, for example, in U.S. Patent Nos. 5,821,332 and 6,156,878, the disclosures of which are incorporated herein by reference in their entireties. In certain embodiments, the anti-OX40 monoclonal antibody is 9B12, or an antigen-binding fragment, variant, or derivative thereof, as described in Weinberg, A.D., et al. J Immunother 29, 575-585 (2006), which is incorporated herein by reference in its entirety.

In certain embodiments of the various aspects disclosed herein, the OX40 agonist is a humanized anti-OX40 antibody or an antigen-binding fragment thereof comprising an antibody VH and an antibody VL, wherein the VL comprises an amino acid sequence at least 70%, 75%, 80%, 85%, 90%, 95%, or 100% identical to the reference amino acid sequence:

DIQMTQSPSSLSASVGDRVTITCRASQDISNYLNWYQQKPGKAPKLLIYYTSKLHSGVPSRF
SGSGSGTDYTLTISSLQPEDFATYYCQQGSALPWTFGQGTKVEIK (SEQ ID NO: 25) or

DIQMTQSPSSLSASVGDRVTITCRASQDISNYLNWYQQKPGKAVKLLIYYTSKLHSGVPSRF
SGSGSRTDYTLTISSLQPEDFATYYCQQGSALPWTFGQGTKVEIK (SEQ ID NO: 26).

In one embodiment, the humanized anti-OX40 antibody or an antigen-binding fragment thereof comprising an antibody VH and an antibody VL, where the VL comprises the amino acid sequence
DIQMTQSPSSLSASVGDRVTITCRASQDISNYLNWYQQKPGKAPKLLIYYTSKLHSGVPSRF SGSGSGTDYTLTISSLQPEDFATYYCQQGSALPWTFGQGTKVEIK (SEQ ID NO: 25) and the VH comprises the amino acid sequence

QVQLQESGPGLVKPSQTLSLTCAVYGGSFSSGYWNWIRKHPGKGLEYIGYISYNGITYHNPS
LKSRITINRDTSKNQYSLQLNSVTPEDTAVYYCARYKYDYDGGHAMDYWGQGTLVTVSS
(SEQ ID NO: 27).

In certain other embodiments the humanized anti-OX40 antibody or an antigen-binding fragment thereof comprises an antibody heavy chain or fragment thereof and an antibody light chain or fragment thereof, where the heavy chain comprises the amino acid sequence disclosed in SEQ ID NO: 28, herein, and the light chain comprises the amino acid sequence disclosed in SEQ ID NO: 29, herein.

In other embodiments, the antibody which specifically binds to OX40, or an antigen-binding fragment thereof binds to the same OX40 epitope as mAb 9B12.

An exemplary humanized OX40 antibody is described by Morris et al., Mol Immunol. May 2007; 44(12): 3112-3121, and has the sequence disclosed in SEQ ID NO: 30, herein. 9B12 is a murine IgG1, anti-OX40 mAb directed against the extracellular domain of human OX40 (CD134) (Weinberg, A.D., et al. J Immunother 29, 575-585 (2006)). It was selected from a panel of anti-OX40 monoclonal antibodies because of its ability to elicit an agonist response for OX40 signaling, stability, and for its high level of production by the hybridoma. For use in clinical applications, 9B12 mAb is equilibrated with phosphate buffered saline, pH 7.0, and its concentration is adjusted to 5.0 mg/ml by diafiltration.

"OX40 ligand" ("OX40L") (also variously termed tumor necrosis factor ligand superfamily member 4, gp34, TAX transcriptionally-activated glycoprotein-1, and CD252) is found largely on antigen presenting cells (APCs), and can be induced on activated B cells, dendritic cells (DCs), Langerhans cells, plamacytoid DCs, and macrophages (Croft, M., (2010) Ann Rev Immunol 28:57-78). Other cells, including activated T cells, NK cells, mast cells, endothelial cells, and smooth muscle cells can express OX40L in response to inflammatory cytokines (Id.). OX40L specifically binds to the OX40 receptor. The human protein is described in U.S. Patent 6,156,878. The mouse OX40L is described in U.S. Patent 5,457,035. OX40L is expressed on the surface of cells and includes an intracellular, a transmembrane and an extracellular receptor-binding domain. A functionally active soluble form of OX40L can be produced by deleting the intracellular and transmembrane domains as described, *e.g*., in U.S. Pat. Nos. 5,457,035; 6,312,700; 6,156,878; 6,242,566; 6,528,055; 6,528,623; 7,098,184; and 7,125,670, the disclosures of which are incorporated herein for all purposes. A functionally active form of OX40L is a form that retains the capacity to bind specifically to OX40, that is, that possesses an OX40 "receptor binding domain." An example is amino acids 51 to 183 of human OX40L. Methods of determining the ability of an OX40L molecule or derivative to bind specifically to OX40 are discussed below. Methods of making and using OX40L and its derivatives (such as derivatives that include an OX40 binding domain) are described in U.S. Pat. Nos. 6,156,878; 6,242,566; 6,528,055; 6,528,623; 7,098,184; and 7,125,670, which also describe proteins comprising the soluble form of OX40L linked to other peptides, such as human immunoglobulin ("Ig") Fc regions, that can be produced to facilitate purification of OX40 ligand from cultured cells, or to enhance the stability of the molecule after *in vivo* administration to a mammal (see also, U.S. Pat. Nos. 5,457,035 and 7,959,925, both of which are incorporated by reference herein in their entireties).

As used herein, the term "OX40L" includes the entire OX40 ligand, soluble OX40 ligand, and functionally active portions of the OX40 ligand. Also included within the definition of OX40L are OX40 ligand variants which vary in amino acid sequence from naturally occurring OX40 ligand molecules but which retain the ability to specifically bind to an OX40 receptor. Such variants are described in U.S. Pat. Nos. 5,457,035; 6,156,878; 6,242,566; 6,528,055; 6,528,623; 7,098,184; and 7,125,670. In a related embodiment, a mutant of OX40L which has lost the ability to specifically bind to OX40, for example amino acids 51 to 183, in which the phenylalanine at position 180 of the receptor-binding domain of human OX40L has been replaced with alanine (F180A) is used.

OX40 agonists include a fusion protein in which one or more domains of OX40L is covalently linked to one or more additional protein domains. Exemplary OX40L fusion proteins that can be used as OX40 agonists are described in U.S. Pat. No. 6,312,700, the disclosure of which is incorporated herein by reference in its entirety. In one embodiment, an OX40 agonist includes an OX40L fusion polypeptide that self-assembles into a multimeric (*e.g*., trimeric or hexameric) OX40L fusion protein. Such fusion proteins are described, *e.g*., in U.S. Patent No. 7,959,925, which is incorporated by reference herein in its entirety. The multimeric OX40L fusion protein exhibits increased efficacy in enhancing antigen specific immune response in a subject, particularly a human subject, due to its ability to spontaneously assemble into highly stable trimers and hexamers.

In another embodiment, an OX40 agonist capable of assembling into a multimeric form includes a fusion polypeptide comprising in an N-terminal to C-terminal direction: an immunoglobulin domain, wherein the immunoglobulin domain includes an Fc domain, a trimerization domain, wherein the trimerization domain includes a coiled coil trimerization domain, and a receptor binding domain, wherein the receptor binding domain is an OX40 receptor binding domain, *e.g*., an OX40L or an OX40-binding fragment, variant, or derivative thereof, where the fusion polypeptide can self-assemble into a trimeric fusion protein. In one aspect, an OX40 agonist capable of assembling into a multimeric form is capable of binding to the OX40 receptor and stimulating at least one OX40 mediated activity. In certain aspects, the OX40 agonist includes an extracellular domain of OX40 ligand.

The trimerization domain of an OX40 agonist capable of assembling into a multimeric form serves to promote self-assembly of individual OX40L fusion polypeptide molecules into a trimeric protein. Thus, an OX40L fusion polypeptide with a trimerization domain self-assembles into a trimeric OX40L fusion protein. In one aspect, the trimerization domain is an isoleucine zipper domain or other coiled coli polypeptide structure. Exemplary coiled coil trimerization domains include: TRAF2 (GENBANK® Accession No. Q12933, amino acids 299-348; Thrombospondin 1 (Accession No. PO7996, amino acids 291-314; Matrilin-4 (Accession No. O95460, amino acids 594-618; CMP (matrilin-1) (Accession No. NP-002370, amino acids 463-496; HSF1 (Accession No. AAX42211, amino acids 165-191; and Cubilin (Accession No. NP-001072, amino acids 104-138. In certain specific aspects, the trimerization domain includes a TRAF2 trimerization domain, a Matrilin-4 trimerization domain, or a combination thereof.

OX40L FP is a human OX40 ligand IgG4P fusion protein that specifically binds to, and triggers signaling by, the human OX40 receptor, a member of the TNFR superfamily. OX40L FP can have the nucleic acid disclosed in any of SEQ ID NOs: 31, 33 and 35 and the correspondingly encoded amino acid sequence disclosed in SEQ ID NO: 32, 34 and 36, respectively. OX40L FP is also disclosed in PCT/US2015/032598, incorporated herein by reference in its entirety. OX40L FP is composed of three distinct domains: (1) human OX40 ligand extracellular receptor binding domains (RBDs) that form homotrimers and bind the OX40 receptor; (2) isoleucine zipper trimerization domains derived from TNFR-associated factor 2 that stabilize the homotrimeric structure of the OX40 ligand RBDs; and (3) human IgG4 fragment crystallizable gamma (Fcγ) domains that facilitate Fcγ receptor clustering of the fusion protein when bound to OX40 receptors, and contain a serine to proline substitution at position 228 (according to EU numbering) in the hinge regions (IgG4P) to promote stability of two sets of OX40 ligand RBD homotrimers. The IgG4P Fc domain is fused directly to an isoleucine zipper trimerization domain derived from amino acid residues 310-349 of human tumor necrosis factor 2 (TRAF2). Fused to the c-terminus of the TRAF2 domain are amino acid residues 51-183 of the extracellular receptor binding domain (RBD) of human OX40L (gene name TNFSF4). The TRAF2 domain stabilizes the homotrimeric structure of OX40L RBDs to enable OX40 binding and activation, while the IgG4P Fc domain confers serum stability, dimerization of OX40L trimers, and facilitates Fcγ receptor clustering of the hexameric fusion protein. One such OX40L FP has the sequences disclosed in SEQ ID NO: 31 and 32. One OX40L FP variant possesses a phenylalanine (F) to alanine (A) mutation at the amino acid corresponding to position 180 in OX40L (SEQ ID NO:33 and 34). Another OX40L FP variant has the IgG4P Fc domain replaced with a human IgG1 Fc domain (SEQ ID NO: 35 and 36). In particular embodiments, the OX40 agonist for use in the present invention is one of the OX40L FP variants.

In particular embodiments, the OX40 agonist for use in the present invention has been modified to increase its serum half-life. For example, the serum half-life of an OX40 agonist can be increased by conjugation to a heterologous molecule such as serum albumin, an antibody Fc region, or PEG. In certain embodiments, OX40 agonists can be conjugated to other therapeutic agents or toxins to form immunoconjugates and/or fusion proteins. In certain embodiments, the OX40 agonist can be formulated so as to facilitate administration and promote stability of the active agent.

### Derivatives

Antibodies for use in the invention (*e.g*., anti-CTLA-4, anti-PD-Ll, anti-PD-1, anti-OX40) may include variants of these sequences that retain the ability to specifically bind their targets. Such variants may be derived from the sequence of these antibodies by a skilled artisan using techniques well known in the art. For example, amino acid substitutions, deletions, or additions, can be made in the FRs and/or in the CDRs. While changes in the FRs are usually designed to improve stability and immunogenicity of the antibody, changes in the CDRs are typically designed to increase affinity of the antibody for its target. Variants of FRs also include naturally occurring immunoglobulin allotypes. Such affinity-increasing changes may be determined empirically by routine techniques that involve altering the CDR and testing the affinity antibody for its target. For example, conservative amino acid substitutions can be made within any one of the disclosed CDRs. Various alterations can be made according to the methods described in Antibody Engineering, 2nd ed., Oxford University Press, ed. Borrebaeck, 1995. These include but are not limited to nucleotide sequences that are altered by the substitution of different codons that encode a functionally equivalent amino acid residue within the sequence, thus producing a "silent" change. For example, the nonpolar amino acids include alanine, leucine, isoleucine, valine, proline, phenylalanine, tryptophan, and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic acid and glutamic acid.
Derivatives and analogs of antibodies of the invention can be produced by various techniques well known in the art, including recombinant and synthetic methods (Maniatis (1990) Molecular Cloning, A Laboratory Manual, 2nd ed., Cold Spring Harbor Laboratory, Cold Spring Harbor, N.Y., and Bodansky et al. (1995) The Practice of Peptide Synthesis, 2nd ed., Spring Verlag, Berlin, Germany). Analogous shuffling or combinatorial techniques are also disclosed by Stemmer (Nature (1994) 370: 389-391), who describes the technique in relation to a β-lactamase gene but observes that the approach may be used for the generation of antibodies.

One may generate novel VH or VL regions carrying one or more sequences derived from the sequences disclosed herein using random mutagenesis of one or more selected VH and/or VL genes. One such technique, error-prone PCR, is described by Gram et al. (Proc. Nat. Acad. Sci. U.S.A. (1992) 89: 3576-3580).

Another method that may be used is to direct mutagenesis to CDRs of VH or VL genes. Such techniques are disclosed by Barbas et al. (Proc. Nat. Acad. Sci. U.S.A. (1994) 91: 3809-3813) and Schier et al. (J. Mol. Biol. (1996) 263: 551-567).

Similarly, one or more, or all three CDRs may be grafted into a repertoire of VH or VL domains, which are then screened for an antigen-binding fragment specific for CTLA-4 or PD-L1.

A portion of an immunoglobulin variable domain will comprise at least one of the CDRs substantially as set out herein and, optionally, intervening framework regions from the scFv fragments as set out herein. The portion may include at least about 50% of either or both of FR1 and FR4, the 50% being the C-terminal 50% of FR1 and the N-terminal 50% of FR4. Additional residues at the N-terminal or C-terminal end of the substantial part of the variable domain may be those not normally associated with naturally occurring variable domain regions. For example, construction of antibodies by recombinant DNA techniques may result in the introduction of N- or C-terminal residues encoded by linkers introduced to facilitate cloning or other manipulation steps. Other manipulation steps include the introduction of linkers to join variable domains to further protein sequences including immunoglobulin heavy chain constant regions, other variable domains (for example, in the production of diabodies), or proteinaceous labels as discussed in further detail below.

A skilled artisan will recognize that antibodies for use in the invention may comprise antigen-binding fragments containing only a single CDR from either VL or VH domain. Either one of the single chain specific binding domains can be used to screen for complementary domains capable of forming a two-domain specific antigen-binding fragment capable of, for example, binding to CTLA-4 and PD-L1.

Antibodies for use in the invention described herein can be linked to another functional molecule, *e.g.,* another peptide or protein (albumin, another antibody, *etc*.). For example, the antibodies can be linked by chemical cross-linking or by recombinant methods. The antibodies may also be linked to one of a variety of nonproteinaceous polymers, *e.g*., polyethylene glycol, polypropylene glycol, or polyoxyalkylenes, in the manner set forth in U.S. Pat. Nos. 4,640,835; 4,496,689; 4,301,144; 4,670,417; 4,791,192; or 4,179,337. The antibodies can be chemically modified by covalent conjugation to a polymer, for example, to increase their circulating half-life. Exemplary polymers and methods to attach them are also shown in U.S. Pat. Nos. 4,766,106; 4,179,337; 4,495,285, and 4,609,546.

The antibodies may also be altered to have a glycosylation pattern that differs from the native pattern. For example, one or more carbohydrate moieties can be deleted and/or one or more glycosylation sites added to the original antibody. Addition of glycosylation sites to the presently disclosed antibodies may be accomplished by altering the amino acid sequence to contain glycosylation site consensus sequences known in the art. Another means of increasing the number of carbohydrate moieties on the antibodies is by chemical or enzymatic coupling of glycosides to the amino acid residues of the antibody. Such methods are described in WO 87/05330, and in Aplin et al. (1981) CRC Crit. Rev. Biochem., 22: 259-306. Removal of any carbohydrate moieties from the antibodies may be accomplished chemically or enzymatically, for example, as described by Hakimuddin et al. (1987) Arch. Biochem. Biophys., 259: 52; and Edge et al. (1981) Anal. Biochem., 118: 131 and by Thotakura et al. (1987) Meth. Enzymol., 138: 350. The antibodies may also be tagged with a detectable, or functional, label. Detectable labels include radiolabels such as 1311 or 99Tc, which may also be attached to antibodies using conventional chemistry. Detectable labels also include enzyme labels such as horseradish peroxidase or alkaline phosphatase. Detectable labels further include chemical moieties such as biotin, which may be detected via binding to a specific cognate detectable moiety, *e.g*., labeled avidin.

Antibodies, in which CDR sequences differ only insubstantially from those set forth herein are encompassed within the scope of this invention. Typically, an amino acid is substituted by a related amino acid having similar charge, hydrophobic, or stereochemical characteristics. Such substitutions would be within the ordinary skills of an artisan. Unlike in CDRs, more substantial changes can be made in FRs without adversely affecting the binding properties of an antibody. Changes to FRs include, but are not limited to, humanizing a non-human derived or engineering certain framework residues that are important for antigen contact or for stabilizing the binding site, *e.g*., changing the class or subclass of the constant region, changing specific amino acid residues which might alter the effector function such as Fc receptor binding, *e.g*., as described in U.S. Pat. Nos. 5,624,821 and 5,648,260 and Lund et al. (1991) J. Immun. 147: 2657-2662 and Morgan et al. (1995) Immunology 86: 319-324, or changing the species from which the constant region is derived.

One of skill in the art will appreciate that the modifications described above are not all-exhaustive, and that many other modifications would be obvious to a skilled artisan in light of the teachings of the present disclosure.

### STAT3 antisense compounds, including antisense oligonucleotides

Antisense compound targeted to STAT3, including antisense oligonucleotides, that specifically bind to and inhibit STAT3 mRNA or protein expression are useful in the present invention.

STAT3 inhibitory antisense oligonucleotides are known in the art.

WO2000/061602 and WO2005/083124 (both Isis Pharmaceuticals Inc.) disclose numerous STAT3 inhibitory antisense oligonucleotides that could feature in the present invention.

WO2012/135736 (Isis Pharmaceuticals Inc.) also discloses numerous STAT3 inhibitory antisense oligonucleotides that could feature in the present invention. Antisense oligonucleotides suitable for use in the embodiments provided herein include, but are not limited to, SEQ ID NOs: 9-426, 430-442, 445-464, 471-498, 500-1034, and 1036-1512 described in WO2012/135736. One of these, identified therein as ISIS 481464, is the molecule also known as AZD9150. It is a gapmer molecule with 3-10-3 configuration, the central gap segment comprising ten 2'-deoxynucleosides, flanked on both sides (in the 5' and 3' directions) by wings comprising three nucleosides each. Each nucleoside in the 5' wing segment and each nucleoside in the 3' wing segment has a cET sugar modification. The internucleotide linkages throughout each gapmer are phosphorothioate (P=S) linkages. All cytosine residues in the gapmer are 5'-methylcytosines. The complete 16-mer nucleobase sequence of AZD9150/ISIS481464 is CTATTTGGATGTCAGC (disclosed herein as SEQ ID NO: 2). Wing segments are underlined. AZD9150 is complementary to nucleobases 3016-3031 in STAT3 sequence of GENBANK Accession No. NM 139276.2, incorporated herein as SEQ ID NO:1.

WO 2014/070868 (Isis Pharmaceuticals Inc.) discloses particular dosages of AZD9150 and methods of treating particular cancers therewith.

AZD9150 is currently being tested in Phase I clinical trials and has shown clinical responses in DLBCL and HCC at tolerated doses.

Other antisense compounds targeted to STAT3 are disclosed in, for example, WO2008109494, which discloses STAT3 meroduplex ribonucleic acid molecules (mdRNAs), and US20100298409, which discloses STAT3 siRNA molecules.

Each of these disclosures is herein incorporated by reference in its entirety.

### Certain embodiments.

In one aspect, the invention provides a method comprising administering to a patient in need thereof an immunomodulatory agent and an antisense compound targeted to STAT3. In one embodiment, the agents ((i) the anti-PD-Ll antibody or antigen-binding fragment thereof; and (ii) the antisense compound targeted to STAT3)) are administered simultaneously, separately or sequentially.

In one aspect, the invention provides a method of treatment that involves administering: (i) at least one immunomodulatory agent; and (ii) an antisense compound targeted to STAT3; to a patient in need thereof. In one embodiment, the agents are administered simultaneously, separately or sequentially.

In one aspect, the invention provides an immunomodulatory agent, and an antisense compound targeted to STAT3, for use in the treatment of a patient in need thereof. In one embodiment, the two agents are administered simultaneously, separately or sequentially.

In one aspect, the invention provides at least one immunomodulatory agent, and an antisense compound targeted to STAT3, for use in the treatment of a patient in need thereof. In one embodiment, the agents are administered simultaneously, separately or sequentially.

In one aspect, the invention provides an immunomodulatory agent for use in the treatment of a patient in need thereof, wherein said patient is also being treated with an antisense compound targeted to STAT3. In one embodiment, the agents are administered simultaneously, separately or sequentially.

In one aspect, the invention provides an antisense compound targeted to STAT3 for use in the treatment of a patient in need thereof, wherein said patient is also being treated with an immunomodulatory agent. In one embodiment, the agents are administered simultaneously, separately or sequentially.

In one aspect, the invention provides the use of an immunomodulatory agent, in the manufacture of a medicament for the treatment of a patient suffering from cancer, which patient is also being treated with an antisense compound targeted to STAT3.

In one aspect, the invention provides the use of an immunomodulatory agent, in the manufacture of a medicament for treating a patient suffering from cancer, wherein the immunomodulatory agent, is administered simultaneously, separately or sequentially with an antisense compound targeted to STAT3, to a patient in need thereof.

In one aspect, the invention provides the use of an antisense compound targeted to STAT3 in the manufacture of a medicament for the treatment of a patient suffering from cancer, which patient is also being treated with an immunomodulatory agent.

In one aspect, the invention provides the use of an antisense compound targeted to STAT3 in the manufacture of a medicament for treating a patient suffering from cancer, wherein the antisense compound targeted to STAT3 is administered simultaneously, separately or sequentially with an immunomodulatory agent.

In one aspect, the invention provides the use of (i) an immunomodulatory agent; and (ii) an antisense compound targeted to STAT3, in the manufacture of a medicament for the treatment of a patient suffering from cancer. In one embodiment, the two agents (antisense compound and immunomodulatory) are administered simultaneously, separately or sequentially.

In one aspect, the invention provides an immunomodulatory agent, for use in the treatment of a patient suffering from cancer, which patient is also being treated with an antisense compound targeted to STAT3.

In one aspect, the invention provides an immunomodulatory agent, for use in the treatment of a patient suffering from cancer, wherein the immunomodulatory agent, is administered simultaneously, separately or sequentially with an antisense compound targeted to STAT3.

In one aspect, the invention provides an antisense compound targeted to STAT3, for use in the treatment of a patient suffering from cancer, which patient is also being treated with an immunomodulatory agent.

In one aspect, the invention provides an antisense compound targeted to STAT3, for use in the treatment of a patient suffering from cancer, wherein the antisense compound targeted to STAT3 is administered simultaneously, separately or sequentially with an immunomodulatory agent.

In one aspect, the invention provides: (i) an immunomodulatory agent; and (ii) an antisense compound targeted to STAT3; for use in the treatment of a patient suffering from cancer, wherein each of the immunomodulatory agent and the antisense compound targeted to STAT3 is administered simultaneously, separately or sequentially to the patient.

In the various aspects disclosed herein, when we refer to treatment of a patient with an immunomodulatory agent it also embraces treatment with more than one immunomodulatory agent.

In particular embodiments of any of the aspects disclosed herein, the immunomodulatory agent is an immune checkpoint inhibitor.

In particular embodiments of any of the aspects disclosed herein, the immunomodulatory agent is: anti-PD-Ll antibody or an antigen-binding fragment thereof.

In particular embodiments of any of the aspects disclosed herein, the immunomodulatory agent is: anti-PD-1 antibody or an antigen-binding fragment thereof.

In particular embodiments of any of the aspects disclosed herein, the immunomodulatory agent is: anti-CTLA-4 antibody or an antigen-binding fragment thereof.

In particular embodiments of any of the aspects disclosed herein, the immunomodulatory agent is: an OX-40 agonist. In particular embodiments the OX-40 agonist is selected from: an anti-OX40 agonist antibody or an antigen-binding fragment thereof, an OX40 ligand or a fragment or derivatives thereof, or an OX40 ligand fusion protein.

In particular embodiments of any of the aspects disclosed herein, the immunomodulatory agent is selected from the group consisting of: MEDI4736, MPDL3280A, BMS936559, 2.7A4, AMP-714, MDX-1105, nivolumab, pembrolizumab, pidilizumab, MPDL3280A, tremelimumab, ipilimumab and OX40L FP.

In one embodiment of any of the aspects disclosed herein the STAT3 is human STAT3, thus the combinations and methods of treatment and the like, utilize an antisense compound targeted to human STAT3.

Certain aspects are drawn to a combination of an antisense compound targeted to STAT3 and one or more immunomodulatory agents. In certain embodiments, such a combination of an antisense compound targeted to STAT3 and one or more immunomodulatory agents comprises AZD9150 and one or more agents selected from: an anti-PD-Ll antibody, an anti-PDl antibody, and anti-CTLA-4 antibody and an OX-40 agonist. In certain embodiments, such a combination comprises AZD9150 as the antisense compound targeted to STAT3 and one or more immunomodulatory agents selected from the group consisting of: MEDI4736, MPDL3280A, BMS936559, 2.7A4, AMP-714, MDX-1105, nivolumab, pembrolizumab, pidilizumab, MPDL3280A, tremelimumab, ipilimumab and OX40L FP.

In particular embodiments of any of the first or second medical uses, or method of treatment aspects disclosed herein, two or more immunomodulatory agents can be administered in combination with the antisense compound targeted to STAT3. For example, the combination treatment may involve a STAT3 ASO, an anti-PDL-1 antibody and an anti-CTLA-4 antibody, such as AZD9150, MEDI4736 and tremelimumab. One alternative would involve a STAT3 ASO, an anti-PDL-1 antibody and an OX40 agonist, such as AZD9150, MEDI4736 and OX40L FP. Another alternative would involve a STAT3 ASO, an anti-PDL-1 antibody and an anti-CTLA-4 antibody, such as AZD9150, MEDI4736 and tremelimumab.

In particular embodiments of any of the first or second medical uses, or method of treatment aspects disclosed herein, the immunomodulatory agent is an anti-PD-Ll antibody or antigen-binding fragment thereof, selected from: MEDI4736, MPDL3280A, BMS936559, 2.7A4, AMP-714 and MDX-1105, or an antigen-binding fragment of any of these.

In particular embodiments of any of the first or second medical uses, or method of treatment aspects above involving an anti-PD-Ll antibody or antigen-binding fragment thereof, the anti-PD-L1 antibody is MEDI4376, or an antigen binding fragment thereof.

In particular embodiments of any of the first or second medical uses, or method of treatment aspects above involving an anti-PD-Ll antibody or antigen-binding fragment thereof, the anti-PD-L1 antibody or antigen binding fragment thereof, blocks the binding of PD-L1 to the PD-1 and CD80 receptors.

In particular embodiments of any of the first or second medical uses, or method of treatment aspects above, the antisense compound targeted to STAT3 is administered to the patient before the immunomodulatory agent. In particular embodiments, the duration between administration of the antisense compound targeted to STAT3 and the immunomodulatory agent, is at least: 10 minutes, 1 hour, 2 hours, 6 hours, 12 hours, 18 hours, 24 hours, 36 hours, 48 hours, 5 days, 7 days 10 days or 14 days. In a particular embodiment, the antisense compound targeted to STAT3 is administered to the patient first and then the immunomodulatory agent is administered later on at a different time point and at a different visitation. A different visitation in this context can mean that the health care provider supplying or administering the immunomodulator drug to the patient does so at a different visit than the visit when the antisense compound targeted to STAT3 was provided/administered. Clearly, the location of the visit is unimportant. The patient could be visiting the healthcare provider or vice versa.

In particular embodiments of any of the first or second medical uses, or method of treatment aspects above, the immunomodulatory agent is administered to the patient before the antisense compound targeted to STAT3.

In particular embodiments, the duration between administration of: (i) the immunomodulatory agent; and, (ii) the antisense compound targeted to STAT3, is at least: 10 minutes, 1 hour, 2 hours, 6 hours, 12 hours, 18 hours, 24 hours, 36 hours, 48 hours, 5 days, 7 days 10 days or 14 days.

In each of the various first or second medical uses, or method of treatment aspects above, the antisense compound targeted to STAT3 can be an antisense oligonucleotide.

In particular embodiments of any of the first or second medical uses, or method of treatment aspects above, the antisense compound targeted to STAT3 can be AZD9150.

In certain embodiments the antisense compound targeted to STAT3 does not inhibit STAT1, STAT4, or STAT6.

In certain embodiments, the patient has cancer. In one embodiment, the patient has a cancer selected from: head and neck cancer (including squamous cell carcinoma (HNSCC)), lymphoma (including diffuse large B-cell carcinoma (DLBCL) or Hodgkin's lymphoma), breast cancer (including triple negative), ovarian cancer (including serous), pancreatic cancer, colorectal cancer, lung cancer (including non small cell lung cancer (NSCLC)), and hepatocellular carcinoma (HCC).

In certain embodiments the cancer cells express PD-L1.

In certain embodiments the patient in need thereof was identified as having a cancer that is PD-L1 positive. In particular embodiments, at least: 20%, 25%, 30%, 40%, 50%, 60%, 70%, 80% or more of the cells in the tumor cells of the patient are PD-L1 positive when assessed using immunochemistry.

In one embodiment, the lung cancer is small cell lung cancer or non-small cell lung cancer (e.g., squamous cell carcinoma, adenocarcinoma, large cell carcinoma, adenosquamous carcinoma and sarcomatoid carcinoma).

In one embodiment, the head and neck cancer is HNSCC.

In one embodiment the lymphoma is DLBCL.

In another aspect, the invention provides a method of treatment involving administering MEDI4736 or an antigen binding fragments thereof, and AZD9150 to a patient identified as having a cancer. In one embodiment of this specific aspect the cancer is selected from: HNSCC, DLBCL, pancreatic cancer, breast cancer, ovarian cancer, NSCLC and HCC.

In another aspect, the invention provides a method of treatment involving administering between about 0.5 - 20 mg/kg/wk of MEDI4736 or an antigen binding fragment thereof, and between about 0.3-5 mg/kg/wk AZD9150, to a patient identified as having cancer. In a particular embodiment, in addition to the AZD9150 and MEDI4736, between about 1-10mg/kg/wk tremelimumab is also administered to the patient. In certain embodiments, the subject's body weight is calculated as the ideal body weight using the Devine formula (Pai, M.P. and Paloucek, F.P. Ann. Pharmacol. 2000. 34: 1066-1069): for men (in kg) = 50 + 2.3 kg/inch over 5 feet; for women (in kg) = 45.5 + 2.3 kg/inch over 5 feet.

In certain embodiments, because of the treatment effects observed, the immunomodulatory agent and/or the antisense compounds targeting STAT3 may be administered at a dose that is lower than the same agent when used as monotherapy. In certain embodiments, the antisense compound targeting STAT3 provided herein is administered to a subject in the range of about 0.3 to 5 milligrams of the antisense compound per kilogram of the subject's body weight per week (0.3-5 mg/kg/wk). Such dose ranges are unexpectedly low for treating cancer. By comparison, a Phase 1 study of LY2275796, an antisense oligonucleotide targeted to cap-binding protein eukaryotic initiation factor 4E (eIF-4E), concluded that the maximum tolerable dose (MTD) and biologically effective dose (BED) of LY2275796 is 1,000 mg under a loading and maintenance dose regimen, but even at a 1,000 mg dose, no tumor response was observed. (Hong D.S. et al., Clin Cancer Res. 2011 17(20):6582-91). In certain embodiments, the immunomodulatory agent is administered to a subject in the range of about 1 to 20 milligrams of the antibody compound per kilogram of the subject's body weight per week (1-20 mg/kg/wk).

In various embodiments of any of the above aspects or any aspect of the invention delineated herein involving an anti-PD-Ll antibody or antigen-binding fragment thereof, the anti-PD-Ll antibody or antigen-binding fragment thereof is selected from: MEDI4736, MPDL3280A, BMS936559, 2.7A4, AMP-714 and MDX-1105, or an antigen-binding fragment of any of these.

In various embodiments of any of the above aspects or any aspect of the invention delineated herein involving an anti-PDl antibody or antigen-binding fragment thereof, the anti-PDl antibody or antigen-binding fragment thereof is selected from: nivolumab, pembrolizumab, pidilizumab and MPDL3280A.

In various embodiments of any of the above aspects or any aspect of the invention delineated herein involving an anti-CTLA-4 antibody or antigen-binding fragment thereof, the CTLA-4 antibody or antigen-binding fragment thereof is selected from: tremelimumab and ipilimumab.

In various embodiments of any of the above aspects or any aspect of the invention delineated herein involving an OX040 agonist, the OX-40 agonist is: OX40L FP.

In various embodiments of any of the above aspects or any aspect of the invention delineated herein the antisense compound targeted to STAT3 is AZD9150.

In certain embodiments, the STAT3 nucleic acid that the antisense compounds can hybridise to is any of the sequences set forth in GENBANK Accession No. NM_139276.2 (incorporated herein as SEQ ID NO: 1) or the complement of GENBANK Accession No. NT_010755.14 truncated from nucleotides 4185000 to 4264000 (referred to as SEQ ID NO:2 in WO2012/135736). In certain embodiments, the antisense oligonucleotide suitable for use herein include, but are not limited to, SEQ ID NOs: 9-426, 430-42, 445-464, 471-498, 500-1034, and 1036-1512 described in WO2012/135736. In certain embodiments the antisense oligonucleotide for use in the present invention comprises a modified oligonucleotide consisting of 12 to 30 linked nucleosides having a nucleobase sequence comprising a portion of at least 12 contiguous nucleobases complementary to an equal length portion of nucleobases 3008 to 3033 of SEQ ID NO: 1, and wherein the nucleobase sequence is complementary to SEQ ID NO: 1. In certain embodiments the antisense oligonucleotide is AZD9150.

Information regarding AZD9150 (aka ISIS 481464), for use in the methods provided herein can be found in WO2012/135736, such as in Example 1, the disclosure of which is incorporated herein by reference in its entirety. AZD9150 is a single stranded modified oligonucleotide comprising ten linked deoxynucleosides (the gap segment), a 5' wing segment consisting of 3 linked nucleosides; and a 3' wing segment consisting of 3 linked nucleosides. The gap segment is positioned between the 5' wing segment and the 3' wing segment and each nucleoside of each wing segment comprises a constrained ethyl nucleoside. Each internucleoside linkage of the oligonucleotide is a phosphorothioate linkage and each cytosine of the oligonucleotide is a 5'-methylcytosine. The complete 16-mer nucleobase sequence of AZD9150 is CTATTTGGATGTCAGC (disclosed herein as SEQ ID NO: 2), wing segments underlined. In certain embodiments, the antisense compound targeted to STAT3 is a compound comprising a modified oligonucleotide consisting of 12 to 30 linked nucleosides and having a nucleobase sequence comprising at least 12 contiguous nucleobases of the nucleobase sequence of SEQ ID NO: 2.

In certain embodiments, the modified oligonucleotide targeting STAT3 consists of a single-stranded modified oligonucleotide.

In certain embodiments, at least one internucleoside linkage of the modified oligonucleotide targeting STAT3 is a modified internucleoside linkage.

In certain embodiments, each internucleoside linkage of the modified oligonucleotide targeting STAT3 is a phosphorothioate internucleoside linkage.

In certain embodiments, at least one nucleoside of the modified oligonucleotide targeting STAT3 comprises a modified sugar.

In certain embodiments, at least one modified sugar of the modified oligonucleotide targeting STAT3 is a bicyclic sugar.

In certain embodiments, the bicyclic sugar of the modified oligonucleotide targeting STAT3 comprises a 4'-CH₂-O-2' bridge or a 4'-CH(CH₃)-O-2' bridge.

In certain embodiments, the modified sugar of the modified oligonucleotide targeting STAT3 comprises a 2'-O(CH₂)₂-OCH₃ group or a 2'-O-CH₃ group.

In certain embodiments, at least one nucleoside of the modified oligonucleotide targeting STAT3 comprises a modified nucleobase. In certain embodiments, the said modified nucleobase is a 5 '-methylcytosine.

In certain embodiments, the modified oligonucleotide targeting STAT3 comprises:
a 5'-wing consisting of 1 to 5 linked nucleosides;
a 3'-wing consisting of 1 to 5 linked nucleosides;
a gap between the 5'-wing and the 3'-wing consisting of 8 to 12 linked 2'-deoxynucleosides; and wherein at least one of the 5'-wing and the 3'-wing comprises at least one bicyclic nucleoside or 2'-substituted nucleoside.

In certain embodiments, the antisense compound targeting STAT3 has a nucleobase sequence that, when written in the 5' to 3' direction, comprises the reverse complement of the target segment of a target nucleic acid to which it is targeted.

It is understood that the sequence set forth in each SEQ ID NO contained herein and as applied to an antisense molecule/compound is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a STAT3 nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds provided herein can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise a chemically modified ribofuranose ring moiety. Examples of chemically modified ribofuranose rings include, without limitation, addition of substitutent groups (including 5' and 2' substituent groups); bridging of non-geminal ring atoms to form bicyclic nucleic acids (BNA); replacement of the ribosyl ring oxygen atom with S, N(R), or C(R1)(R)2 (R = H, C₁-C₁₂ alkyl or a protecting group); and combinations thereof. Examples of chemically modified sugars include, 2'-F-5'-methyl substituted nucleoside (*see,* PCT International Application WO 2008/101157 for other disclosed 5', 2'-bis substituted nucleosides), replacement of the ribosyl ring oxygen atom with S with further substitution at the 2'-position (*see,* published U.S. Patent Application US2005/0130923), or, alternatively, 5'-substitution of a BNA (*see,* PCT International Application WO 2007/134181, wherein LNA is substituted with, for example, a 5'-methyl or a 5'-vinyl group).

Examples of nucleosides having modified sugar moieties include, without limitation, nucleosides comprising 5'-vinyl, 5'-methyl (R or S), 4'-S, 2'-F, 2'-OCH₃, and 2'-O(CH₂)2OCH₃ substituent groups. The substituent at the 2' position can also be selected from allyl, amino, azido, thio, O-allyl, O-C₁-C₁₀ alkyl, OCF₃, O(CH₂)2SCH₃, O(CH₂)2-O-N(Rm)(Rn), and O-CH₂-C(=O)-N(Rm)(Rn), where each Rm and Rn is, independently, H or substituted or unsubstituted C₁-C₁₀ alkyl.

### Conjugated Antisense compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

### Formulations

The immunomodulatory agent(s) and the antisense compound targeted to a STAT3 nucleic acid can be utilized in pharmaceutical compositions by combining the compound(s) with a suitable pharmaceutically acceptable diluent or carrier. A pharmaceutically acceptable diluent includes phosphate-buffered saline (PBS). Suitable examples of carriers include physiological saline, polyethylene glycol, ethanol, vegetable oils, isopropyl myristate, etc., but are not limited to them.

Acceptable carriers or diluents for therapeutic use are well known in the pharmaceutical art, and are described, for example, in Remington's Pharmaceutical Sciences, Mack Publishing Co., A.R Gennaro edit., 1985.

Pharmaceutical compositions comprising the immunomodulatory agent and/or the antisense compounds encompass any pharmaceutically acceptable salts, esters, or salts of such esters which, upon administration to an animal, including a human, is capable of providing (directly or indirectly) the biologically active metabolite or residue thereof.

In certain embodiments the two agents (immunomodulatory agent and the STAT3 antisense compound) are formulated separately.

According to one aspect there is provided a pharmaceutical composition comprising an immunomodulatory agent and an antisense compound targeted to STAT3 and one or more pharmaceutically acceptable carriers or diluent.

The agents of the present invention may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as solutions, suspensions, or emulsions in oily or aqueous vehicles, for example solutions in aqueous polyethylene glycol. Examples of oily or nonaqueous carriers, diluents, or vehicles include propylene glycol, polyethylene glycol, vegetable oils (e.g., olive oil), and injectable organic esters (e.g. , ethyl oleate). The compositions may also contain other formulatory agents such as wetting, emulsifying or suspending, preserving, stabilizing and/or dispersing agents. Alternatively, the active ingredient may be in powder form, obtained by aseptic isolation of sterile solid or by lyophilization from solution for constitution before use with a suitable vehicle, e.g., sterile, pyrogen-free water.

In one embodiment, the anti-PD-Ll antibody MEDI4736 is formulated at 50 mg/mL in 26 mM histidine/histidine-HCl, 275 mM trehalose dihydrate, 0.02% (weight/volume [w/v]) polysorbate 80, pH 6.0. The product can then be supplied as a white to off-white lyophilized powder in clear glass vials (e.g. 10R vial), e.g. closed with an elastomeric stopper and a flip-off cap overseal. Each vial contains 200 mg (nominal) of active product. MEDI4736 is then reconstituted using aseptic techniques with 4.0 mL sterile water for injection (WFI) to give a final concentration of 50 mg/mL. The reconstituted solution is then diluted with 0.9% (w/v) saline for IV infusion using, for example, syringes or bags.

### Treatment regimes.

In various embodiments of any of the above aspects, the treatment is administered every 1, 2, 3 or 4 weeks. In various embodiments, the patient is first administered one or a series of loading dose(s) of the immunomodulatory agent (e.g. anti-PD-Ll antibody MEDI4736). This could entail dosing the agent multiple times in the early stage of treatment (loading phase), e.g. on days 1, 3 and 5 in week 1.

The inventors have found that treating the patient with the STAT3 inhibitor first followed later by the immunomudulator yield better anti-cancer results. In various embodiments, the patient is first administered one or a series of loading dose(s) of the antisense compound targeted to a STAT3 nucleic acid (e.g. AZD9150). This could entail dosing the agent multiple times in the early stage of treatment, e.g. on days 1, 3 and 5 in week 1.

The purpose of a loading dose phase is to reach steady state or efficacious level of agent (as measured in blood) more quickly, before the "maintenance" phase of treatment, e.g. once a week.

Pretreatment with the STAT3 selective inhibitor may also serve to provide an increase in tumor infiltrating CD45+ cells, and possibly also a decrease in tumor infiltrating macrophages, providing a tumor environment that is more susceptible to effective treatment with an immunomodulatory agent.

In various embodiments of any of the above aspects between about 1mg/kg and 20mg/kg, inclusive, of an immunomodulatory agent, and between about 1mg/kg and 15mg/kg, inclusive, of an antisense compound targeted to a STAT3 nucleic acid is administered to a patient.

In various embodiments of any of the above aspects involving MEDI4736 or an antigen-binding fragment thereof, between about 2mg/kg and 10mg/kg, inclusive, of MEDI4736 or an antigen-binding fragment thereof, and between about 1mg/kg and 3mg/kg, inclusive, AZD9150 is administered. In various embodiments of any of the above aspects involving MEDI4736 or an antigen-binding fragment thereof, about 1 mg/kg MEDI4736 and about 1 mg/kg AZD9150 is administered, about 1 mg/kg MEDI4736 and about 2 mg/kg AZD9150 is administered, about 1 mg/kg MEDI4736 and about 3 mg/kg AZD9150 is administered; about 1 mg/kg MEDI4736 and about 10 mg/kg AZD9150 is administered, about 3 mg/kg MEDI4736 and about 1 mg/kg AZD9150 is administered; about 3 mg/kg MEDI4736 and about 2 mg/kg AZD9150 is administered; about 3 mg/kg MEDI4736 and about 3 mg/kg AZD9150 is administered; about 3 mg/kg MEDI4736 and about 10 mg/kg AZD9150 is administered; about 10 mg/kg MEDI4736 and about 1 mg/kg AZD9150 is administered; about 10 mg/kg MEDI4736 and about 2 mg/kg AZD9150 is administered; about 10 mg/kg MEDI4736 and about 3 mg/kg AZD9150 is administered; about 10 mg/kg MEDI4736 and about 10 mg/kg AZD9150 is administered, about 15 mg/kg MEDI4736 and about 1 mg/kg AZD9150 is administered; about 15 mg/kg MEDI4736 and about 2 mg/kg AZD9150 is administered; about 15 mg/kg MEDI4736 and about 3 mg/kg AZD9150 is administered or about 15 mg/kg MEDI4736 and about 10 mg/kg AZD9150 is administered. In certain embodiments, the subject's body weight is calculated as the ideal body weight using the Devine formula.

In various embodiments of any of the above aspects, the method results in an increase in overall survival (e.g., an increase of weeks, months or years) as compared to the administration of either the immunomodulatory agent (e.g. MEDI4736 or an antigen-binding fragment thereof) or the AZD9150 alone. In particular, the increase in survival is more than about 4-6 weeks, 1-2 months, 3-4 months, 5-7 months, 6-8 months, or 9-12 months. In various embodiments of any of the above aspects, the administration of the immunomodulatory agent (e.g. MEDI4736 or an antigen-binding fragment thereof) is repeated about every 4 weeks. In various embodiments of any of the above aspects, the administration of AZD9150 is repeated about every 4 weeks. In various embodiments of any of the above aspects, the administration of AZD9150 is repeated about every 12 weeks. In various embodiments of any of the above aspects, the administration of AZD9150 is administered about every 4 weeks for seven administrations and then every 12 weeks. In various embodiments of any of the above aspects, the administration of the immunomodulatory agent (e.g. MEDI4736 or an antigen-binding fragment thereof), is by intravenous infusion. In various embodiments of any of the above aspects, the administration of AZD9150 is by intravenous infusion. In various embodiments of any of the above aspects, AZD9150 and MEDI4736 or an antigen-binding fragment thereof, are administered concurrently or at different times. In various embodiments of any of the above aspects, AZD9150 and MEDI4736 or an antigen-binding fragment thereof, are administered twenty-four, forty-eight or seventy-two hours apart, 1, 2, or 3 weeks apart, or between 1, 2, and 3 months apart.

Combinations involving three agents are also contemplated. In particular embodiments of any of the aspects disclosed herein, AZD9150, MEDI4736 and tremelimumab are used in the combination and/or treatments. Particular dosage for these agents include, AZD9150 at 2mg/kg or 3mg/kg, MEDI4736 at 5mg/kg, 10mg/kg or 20mg/kg and tremelimumab at 1mg/kg or 2mg/kg.

Other features and advantages of the invention will be apparent from the detailed description, and from the claims.

In certain aspects, a patient presenting with a cancer/tumor is administered: (i) at least one immunomodulatory agent; and, (ii) an antisense compound targeted to STAT3; in pharmaceutically effective amounts.

In certain aspects, a patient presenting with a cancer/tumor is administered: (i) MEDI4736 or an antigen-binding fragment thereof; and, (ii) AZD9150; in pharmaceutically effective amounts. In a particular embodiment of this aspect, the patient is also administered tremelimumab, or an antigen binding fragment thereof. MEDI4736 or an antigen-binding fragment thereof, and AZD9150 can be administered only once or infrequently while still providing benefit to the patient. In further aspects the patient is administered additional follow-on doses. Follow-on doses can be administered at various time intervals depending on the patient's age, weight, clinical assessment, tumor burden, and/or other factors, including the judgment of the attending physician.

The intervals between doses of the immunomodulatory agent (e.g. MEDI4736 or an antigen-binding fragment thereof and/or tremelimumab or an antigen-binding fragment thereof), and of the antisense compound targeted to STAT3 (e.g. AZD9150) can be every one, two, three, four, five or six weeks. The intervals between doses of MEDI4736, or an antigen-binding fragment thereof, and AZD9150 can be every four weeks for six cycles and then every twelve weeks. In certain aspects, MEDI4736 or an antigen-binding fragment thereof, is administered about twice as frequently as AZD9150. In certain aspects, MEDI4736 or an antigen-binding fragment thereof, is administered about six times as frequently as AZD9150. In certain aspects, AZD9150 is administered about twice as frequently as MEDI4736 or an antigen-binding fragment thereof. In certain aspects, AZD9150 is administered about six times as frequently as MEDI4736 or an antigen-binding fragment thereof.

In some embodiments, at least three doses, at least four doses, at least five doses, at least six doses, at least seven doses, at least eight doses, at least nine doses, at least ten doses, or at least fifteen doses or more of each agent can be administered to the patient. In some embodiments, the immunomodulatory agent (e.g. MEDI4736 or an antigen-binding fragment thereof), is administered over a two-week treatment period, over a four-week treatment period, over a six-week treatment period, over an eight-week treatment period, over a twelve-week treatment period, over a twenty-four-week treatment period, or over a one-year or more treatment period. In some embodiments, AZD9150 is administered over a two-week treatment period, a four-week treatment period, over an eight-week treatment period, over a twelve-week treatment period, over a twenty-four-week treatment period, or over a one-year or more treatment period

The amount of (i) immunomodulatory agent, and (ii) antisense compound targeted to STAT3 to be administered to an individual patient may depend on various parameters such as the patient's age, weight, clinical assessment, tumor burden and/or other factors, including the judgment of the attending physician. The amount of the immunomodulatory agent (e.g. anti-PD-Ll antibody or antigen-binding fragment thereof), and antisense compound targeted to a STAT3 nucleic acid (e.g. AZD9150) to be administered to the patient may be determined from comprehensive clinical trials of the agents in the patient population.

In certain embodiments involving the anti-PD-Ll antibody MED14736, the patient is administered one or more doses of MEDI4736 or an antigen-binding fragment thereof, wherein the dose is about 0.3 mg/kg. In certain embodiments the patient is administered one or more doses of MEDI4736 or an antigen-binding fragment thereof, wherein the dose is about 1 mg/kg. In certain embodiments the patient is administered one or more doses of MEDI4736 or an antigen-binding fragment thereof, wherein the dose is about 3 mg/kg. In certain embodiments the patient is administered one or more doses of MEDI4736 or an antigen-binding fragment thereof, wherein the dose is about 10 mg/kg. In certain embodiments the patient is administered one or more doses of MEDI4736 or an antigen-binding fragment thereof, wherein the dose is about 15 mg/kg. In certain embodiments the patient is administered one or more doses of MEDI4736 or an antigen-binding fragment thereof, wherein the dose is about 20 mg/kg. In certain embodiments each of the doses of MEDI4736 or an antigen-binding fragment thereof, are administered at least one week apart, such as qw, 2qw, 3qw, 4qw, 8qw and 12qw. In certain embodiments, the subject's body weight is calculated as the ideal body weight using the Devine formula.

In certain embodiments involving the antisense compound targeted to STAT3 known as AZD9150, the patient is administered one or more doses of AZD9150 wherein the dose is about 0.3 mg/kg. In certain embodiments the patient is administered one or more doses of AZD9150 wherein the dose is about 1 mg/kg. In certain embodiments the patient is administered one or more doses of AZD9150 wherein the dose is about 3 mg/kg. In certain embodiments the patient is administered one or more doses of AZD9150 wherein the dose is about 10 mg/kg. In certain embodiments the patient is administered one or more doses of AZD9150 wherein the dose is about 15 mg/kg. In certain embodiments the patient is administered one or more doses of AZD9150 wherein the dose is about 20 mg/kg. In certain embodiments each of the doses of AZD9150 are administered at least one week apart. In certain embodiments each of the doses of AZD9150 are administered 2 week apart (2QW). In certain embodiments each of the doses of AZD9150 are administered 3 week apart (3QW). In certain embodiments each of the doses of AZD9150 are administered 4 week apart (4QW). In certain embodiments each of the doses of AZD9150 are administered 8 week apart (8QW). In certain embodiments each of the doses of AZD9150 are administered 12 week apart (12QW).

In certain embodiments involving the anti-CTLA-4 antibody tremelimumab or ipilimumab, or an antigen-binding fragment of either, the patient is administered one or more doses of the anti-CTLA-4 antibody or an antigen-binding fragment thereof wherein the dose is about 1 mg/kg. In certain embodiments the patient is administered one or more doses of the anti-CTLA-4 antibody or an antigen-binding fragment thereof wherein the dose is about 3 mg/kg. In certain embodiments the patient is administered one or more doses of the anti-CTLA-4 antibody or an antigen-binding fragment thereof wherein the dose is about 10 mg/kg.

In certain embodiments the patient is administered at least two doses of the anti-CTLA-4 antibody or an antigen-binding fragment thereof wherein the dose is about 1 mg/kg. In certain embodiments the patient is administered at least two doses of the anti-CTLA-4 antibody or an antigen-binding fragment thereof wherein the dose is about 3 mg/kg. In certain embodiments the patient is administered at least two doses of the anti-CTLA-4 antibody or an antigen-binding fragment thereof wherein the dose is about 10 mg/kg. In some embodiments, the at least two doses are administered about four weeks apart. In some embodiments, the at least two doses are administered about twelve weeks apart.

In certain embodiments the patient is administered at least three doses of the anti-CTLA-4 antibody or an antigen-binding fragment thereof wherein the dose is about 1 mg/kg. In certain embodiments the patient is administered at least three doses of the anti-CTLA-4 antibody or an antigen-binding fragment thereof wherein the dose is about 3 mg/kg. In certain embodiments the patient is administered at least three doses of the anti-CTLA-4 antibody or an antigen-binding fragment thereof wherein the dose is about 10 mg/kg. In some embodiments, the at least three doses are administered about four weeks apart. In some embodiments, the at least three doses are administered about twelve weeks apart.

In certain embodiments the patient is administered at least two doses of MEDI4736 or an antigen-binding fragment thereof, wherein the dose is about 1 mg/kg. In certain embodiments the patient is administered at least two doses of MEDI4736 or an antigen-binding fragment thereof, wherein the dose is about 3 mg/kg. In certain embodiments the patient is administered at least two doses of MEDI4736 or an antigen-binding fragment thereof, wherein the dose is about 10 mg/kg. In certain embodiments the patient is administered at least two doses of MEDI4736 or an antigen-binding fragment thereof, wherein the dose is about 15 mg/kg. In certain embodiments the patient is administered at least two doses of MEDI4736 or an antigen-binding fragment thereof, wherein the dose is about 20 mg/kg. In certain embodiments the patient is administered at least two doses of AZD9150 wherein the dose is about 1 mg/kg. In certain embodiments the patient is administered at least two doses of AZD9150 wherein the dose is about 2 mg/kg. In certain embodiments the patient is administered at least two doses of AZD9150 wherein the dose is about 3 mg/kg. In certain embodiments, the subject's body weight is calculated as the ideal body weight using the Devine formula.

In some embodiments, the at least two doses are administered about four weeks apart. In some embodiments, the at least two doses are administered about twelve weeks apart.
In certain embodiments, administration of the agents, such as MEDI4736 or an antigen-binding fragment thereof, and/or AZD9150, according to the methods provided herein is through parenteral administration. For example, one or both agents can be administered by intravenous infusion or by subcutaneous injection. In some embodiments, the administration is by intravenous infusion.

Effective treatment with a combination of an immunomodulator (such as anti-CTLA-4 antibody, anti-PD-Ll antibody, anti-PD-1 antibody, and OX40 agonist) and an antisense compound targeted to STAT3 (such as AZD9150) includes, for example, reducing the rate of progression of the cancer, retardation or stabilization of tumor or metastatic growth, tumor shrinkage, and/or tumor regression, either at the site of a primary tumor, or in one or more metastases. In some aspects the reduction or retardation of tumor growth can be statistically significant. A reduction in tumor growth can be measured by comparison to the growth of patient's tumor at baseline, against an expected tumor growth, against an expected tumor growth based on a large patient population, or against the tumor growth of a control population. In other embodiments, the methods of the invention increase survival.

Clinical response to administration of an immunomodulator (such as anti-CTLA-4 antibody, anti-PD-Ll antibody, anti-PD-1 antibody, and OX40 agonist) and an antisense compound targeted to STAT3 (such as AZD9150) can be assessed using diagnostic techniques known to clinicians, including but not limited to magnetic resonance imaging (MRI) scan, x-radiographic imaging, computed tomographic (CT) scan, flow cytometry or fluorescence-activated cell sorter (FACS) analysis, histology, gross pathology, and blood chemistry, including but not limited to changes detectable by ELISA, RIA, and chromatography.

The methods provided herein can decrease or retard cancer tumor growth. In some cases the reduction or retardation can be statistically significant. A reduction in tumor growth can be measured by comparison to the growth of patient's tumor at baseline, against an expected tumor growth, against an expected tumor growth based on a large patient population, or against the tumor growth of a control population.

In certain embodiments, administering the dose of an antisense compound targeted to STAT3 and an immunomodulatory agent reduces tumor size or tumor volume in the subject. In certain embodiments, administering the dose of the antisense compound and the immunomodulatory agent prolongs survival of the subject. In certain embodiments, administering the dose of the antisense compound targeted to a STAT3 and the immunomodulatory agent, treats cancer, such as B-cell lymphoma, in the subject. In certain embodiments, the method is effective to treat cancer and acceptably tolerable in a subject.

In certain embodiments, a tumor response is measured using the Immune-related Response Criteria (irRc). In certain embodiments, a tumor response is measured using the Response Evaluation Critera in Solid Tumors (RECIST).

In certain embodiments, a tumor response is detectable at week 7 or thereafter, such as at week 13, at week 25, at week 41, at week 52.

In certain embodiments, a patient achieves disease control (DC). Disease control can be a complete response (CR), partial response (PR), or stable disease (SD).

A "complete response" (CR) refers to the disappearance of all lesions, whether measurable or not, and no new lesions. Confirmation can be obtained using a repeat, consecutive assessment no less than four weeks from the date of first documentation. New, non-measurable lesions preclude CR.

A "partial response" (PR) refers to a decrease in tumor burden ≥ 30% relative to baseline. Confirmation can be obtained using a consecutive repeat assessment at least 4 weeks from the date of first documentation.

"Stable disease" (SD) indicates a decrease in tumor burden of less than about 30% relative to baseline cannot be established and a 20% or greater increase compared to nadir cannot be established.

In certain embodiments, administration of the immunomodulatory agent and the antisense compound targeted to STAT3 can increase progression-free survival (PFS).

In certain embodiments, administration of the immunomodulatory agent and the antisense compound targeted to STAT3 can increase overall survival (OS).

In certain embodiments, administration of MEDI4736 or an antigen-binding fragment thereof, and AZD9150 can increase progression-free survival (PFS).

In certain embodiments, administration of MEDI4736 or an antigen-binding fragment thereof, and AZD9150 can increase overall survival (OS).

In some embodiments, the patient has previously received treatment with at least one chemotherapeutic agent. In some embodiments, the patient has previously received treatment with at least two chemotherapeutic agents. The chemotherapeutic agent can be, for example, and without limitation, Vemurafenib, Gefitinib, Erlotinib, Afatinib, Cetuximab, Carboplatin, Bevacizumab, and/or Pemetrexed.

In some embodiments, the cancer is refractory or resistant to at least one chemotherapeutic agent. In some embodiments, the cancer is refractory or resistant to at least two chemotherapeutic agents. The cancer can be refractory or resistant to one or more of, for example, and without limitation, Vemurafenib, Gefitinib, Erlotinib, Afatinib, Cetuximab, Carboplatin, Bevacizumab, and/or Pemetrexed.

In some embodiments, the patient has an Eastern Cooperative Oncology Group (ECOG) (Oken MM, et al. Am. J. Clin. Oncol. 5: 649-55 (1982)) performance status of 0, 1, or 2 prior to the administration of MEDI4736 or an antigen-binding fragment thereof, and AZD9150.

As provided herein, MEDI4736 or an antigen-binding fragment thereof, can also decrease free (soluble) PD-L1 levels. Free (soluble) PD-L1 refers to PD-L1 that is not bound (e.g., by MEDI4736). In some embodiments, sPD-L1 levels are reduced and/or undetectable following administration of MEDI4736 or an antigen-binding fragment thereof, and AZD9150. In some embodiments, administration of MEDI4736 or an antigen-binding fragment thereof, and AZD9150 reduces the rate of increase of free (soluble) PD-L1 levels as compared, e.g., to the rate of increase of free (soluble) PD-L1 levels prior to the administrations.

Treatment of a patient with a cancer using both (i) an immunomodulatory agent, such as MEDI4736, or an antigen-binding fragment thereof; and, (ii) an antisense compound targeted to STAT3, such as AZD9150, (i.e., co-therapy) as provided herein can result in an additive and/or synergistic effect. As used herein, the term "synergistic" refers to a combination of therapies (e.g., a combination of MEDI4736 or an antigen-binding fragment thereof, and AZD9150) which is more effective than the additive effects of the single therapies.

A synergistic effect of a combination of therapies (e.g., a combination of a MEDI4736 or an antigen-binding fragment thereof, and AZD9150) may permit the use of lower dosages of one or more of the therapeutic agents and/or less frequent administration of said therapeutic agents to a patient with cancer. The ability to utilize lower dosages of therapeutic agents and/or to administer said therapies less frequently reduces the toxicity associated with the administration of said therapies to a subject without reducing the efficacy of said therapies in the treatment of a cancer. In addition, a synergistic effect can result in improved efficacy of therapeutic agents in the management, treatment, or amelioration of a cancer. The synergistic effect of a combination of therapeutic agents can avoid or reduce adverse or unwanted side effects associated with the use of either single therapy. The synergistic effect of a combination of therapeutic agents may also manifest itself as a reduction in tumor mass (or tumor regression). The synergistic effect of a combination of therapeutic agents may also manifest itself as a sustained reduction in tumor growth rate.

In co-therapy, the immunomodulatory agent (such as MEDI4736 or an antigen-binding fragment thereof), can be optionally included in the same pharmaceutical composition as the antisense compound targeted to STAT3 (such as AZD9150), or may be included in a separate pharmaceutical composition. In this latter case, the pharmaceutical composition comprising the immunomodulatory agent, is suitable for administration prior to, simultaneously with, or following administration of the pharmaceutical composition comprising the antisense compound targeted to STAT3. In certain instances, the the immunomodulatory agent, is administered at overlapping times as the antisense compound targeted to STAT3 in a separate composition.

### Kits

In another aspect, the invention provides kits for treating cancer comprising: (i) an antisense compound targeted to STAT3 (such as AZD9150); and, (ii) an immunomodulatory agent (such as an anti-PD-Ll antibody or an antigen-binding fragment thereof). In one embodiment, the kit includes a therapeutic composition comprising the immunomodulatory agent (such as an anti-PD-Ll antibody or an antigen-binding fragment thereof), and the antisense compound targeted to STAT3 (such as AZD9150), each in unit dosage form. In certain embodiments the antisense compound targeted to STAT3 can be AZD9150 and/or the immunomodulatory agent, can be selected from the group consisting of: MEDI4736, MPDL3280A, BMS936559, 2.7A4, AMP-714, MDX-1105, nivolumab, pembrolizumab, pidilizumab, MPDL3280A, tremelimumab, ipilimumab and OX40L FP.

When the immunomodulatory agent is an anti-PD-Ll antibody, it can be selected from: MEDI4736, MPDL3280A, BMS936559, 2.7A4, AMP-714 and MDX-1105, or an antigen-binding fragment of any of these.

In some embodiments, the kit comprises a sterile container which contains one or more therapeutic compositions; such containers can be boxes, ampoules, bottles, vials, tubes, bags, pouches, blister-packs, or other suitable container forms known in the art. Such containers can be made of plastic, glass, laminated paper, metal foil, or other materials suitable for holding medicaments.

If desired, the kit further comprises instructions for administering the immunomodulatory agent (e.g. anti-PD-Ll antibody or an antigen-binding fragment thereof), and the antisense compound targeted to STAT3 (e.g. AZD9150) to a subject having a cancer. In particular embodiments, the instructions include at least one of the following: description of the therapeutic agent(s); dosage schedule and administration for treatment or prevention of cancer or symptoms thereof; precautions; warnings; indications; counter-indications; over dosage information; adverse reactions; animal pharmacology; clinical studies; and/or references. The instructions may be printed directly on the container (when present), or as a label applied to the container, or as a separate sheet, pamphlet, card, or folder supplied in or with the container.

In another aspect, there is provided a product containing an antisense compound targeted to STAT3 and an immunomodulatory agent, as a combined preparation for simultaneous, separate or sequential use in treating cancer.

In such a product the antisense compound targeted to STAT3 is or can be AZD9150 and/or the immunomodulatory agent is or can be selected from: MEDI4736, MPDL3280A, BMS936559, 2.7A4, AMP-714, MDX-1105, nivolumab, pembrolizumab, pidilizumab, MPDL3280A, tremelimumab, ipilimumab and OX40L FP.

In other embodiment, in such a product the antisense compound targeted to STAT3 is or can be AZD9150 and/or the anti-PD-Ll antibody or an antigen-binding fragment thereof, is or can be selected from: MEDI4736, MPDL3280A, BMS936559, 2.7A4, AMP-714 and MDX-1105, or an antigen-binding fragment of any of these.

The practice of the present invention employs, unless otherwise indicated, conventional techniques of molecular biology (including recombinant techniques), microbiology, cell biology, biochemistry immunohistochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan et al., 1991). These techniques are applicable to the production of the polynucleotides and polypeptides of the invention, and, as such, may be considered in making and practicing the invention. Particularly useful techniques for particular embodiments will be discussed in the sections that follow.

The following examples and figures are put forth so as to provide those of ordinary skill in the art with a complete disclosure and description of how to make and use the various aspects of the invention, and are not intended to limit the scope of what the inventors regard as their invention.

### Figure Legend

- Figure 1: Tumor infiltrating leukocytes in CT-26 tumors from mice treated with muSTAT3 ASO (% of total live cells, relative to vehicle control).
- Figure 2: CT-26 tumor volumes (mean +/- SEM (2a) and individual mice (2b) and (2c)) after treatment with mouse STAT3 ASO as a single agent and in combination with an antibody targeting PD-L1.
- Figure 3: CT-26 tumor volumes after treatment with mouse STAT3 ASO as a single agent and in combination with PD-L1 Ab, CTLA-4 Ab, or OX4- ligand fusion protein.
- Figure 4: CT-26 tumor volumes after treatment with AZD9150, PD-L1 Ab, or isotype control Ab as single agents, or a combination of AZD9150 plus PD-L1 Ab.
- Figure 5: Tumor infiltrating lymphocytes in CT-26 tumors from mice treated with a JAK1 selective inhibitor (% of total live cells, relative to vehicle control).
- Figure 6: CT-26 tumor volumes after treatment with AZ-JAK1, anti-PD-Ll Ab, or combination of the two treatments. Mean ± SEM.
- Figure 7: STAT3 mRNA levels in tumor and blood samples from CT-26 tumor bearing mice after treatment with STAT3 ASO, anti-PD-Ll Ab, or a combination of the two treatments. Mean +/- SEM of 4 samples from each treatment group.

### Examples:

### Example 1. Preclinical studies showing that STAT3 inhibition using ASO in mouse bolsters the immune system.

### Antisense oligos used:

Both ASOs used are 3-10-3 gapmers with cET chemistry and phosphorothioate linkages, all bases are 2'-deoxynucleosides. Wing portions are underlined.
*Mouse STAT3 ASO: GAA*ATTCATTCTT*CCA* [SEQ ID NO: 11)
*Control ASO: GGC*TACTACGCCG*TCA* (SEQ ID NO: 12)

Oligos can be synthesized according to standard techniques, such as that described in Seth et al., (J Med Chem. 52(1): 10-13, 2009).

CT-26 mouse colon tumor cells (5 x 10⁵ / mouse) were implanted subcutaneously in female BALB/c mice. The mice were randomized into groups of 8 (based on body weight), and treated with either PBS vehicle, non-targeting control antisense oligonucleotide (ASO) SEQ ID NO: 12], or mouse STAT3 targeted ASO (SEQ ID NO: 11) at 50 mg/kg. All ASOs were formulated in PBS and dosed subcutaneously at 50 mg/kg, QD, on a 5 days on / 2 days off schedule, beginning 2 days after tumor implantation. At 12 and 26 days after initiation of dosing, 4 mice from each group were selected and tumors harvested and processed for flow cytometry analysis. Cells from the tumors harvested at day 12 (week 2) were pooled prior to analysis, due to small tumor size, while tumors harvested at day 26 (week 4) were analysed individually. CD45+ cells were quantified at weeks 2 and 4. Macrophages were quantified at week 4. The various cells were identified and quantified by immunostaining and flow cytometry analysis using a BD FACS Canto II flow cytometer and FlowJo software.

The practice of the present invention employs, unless otherwise indicated, conventional techniques in cell biology, immunohistochemistry and immunology, which are well within the purview of the skilled artisan. Such techniques are explained fully in the literature, such as "Handbook of Experimental Immunology" (Weir, 1996) and "Current Protocols in Immunology" (Coligan et al., 1991).

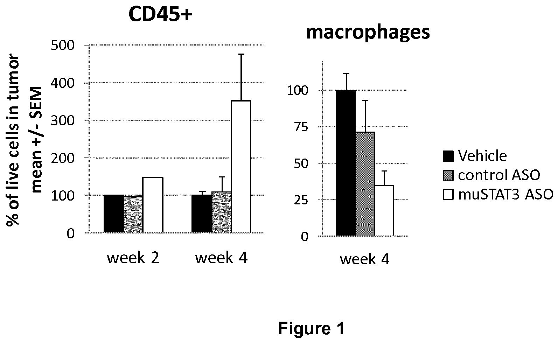

Treatment with the STAT3 ASO resulted in changes in the tumor immune infiltrate, relative to vehicle and control ASO treated mice (Figure 1). An increase in total immune infiltrate (CD45+ leukocytes) in the mouse STAT3 ASO treated group, relative to the vehicle and control ASO treated groups, was observed at weeks 2 (149%) and 4 (353%). Macrophages (CD45+F4/80+) were lower (65% decrease) in tumors at week 4.

The elevation in tumor infiltrating CD45+ cells, along with the decrease in tumor infiltrating macrophages, indicate immune modulation and enhanced antitumor immune response associated with STAT3 ASO treatment.

### Example 2. Pretreatment vs. concurrent treatment with mouse STAT3 ASO in combination with anti-PD-Ll antibody treatment provides enhanced antitumor activity.

CT-26 mouse colon tumor cells (5 x 10⁵ / mouse) were implanted subcutaneously in female BALB/c mice. Mice were treated with either PBS vehicle, mouse STAT3 ASO (same as used in Example 1), an anti-PD-Ll antibody (anti-mouse PD-L1, clone 10F.9G2, rat IgG2b isotype, purchased from BioXCell, West Lebanon, NH), an isotype control Ab (rat IgG2b, product LTF-2, purchased from BioXCell, West Lebanon NH), or a combination of mouse STAT3 ASO + PD-L1 antibody. ASO treatments began either before randomization into treatment groups (day 2 after implant), or at the time of randomization (day 9 after implant). At the time of randomization, the tumor volume in mice receiving ASO treatment was not significantly different than the tumor volume in mice that had not received ASO treatment. There were ten mice in each treatment group. The ASOs were formulated in PBS and dosed subcutaneously. The Abs were also formulated in PBS, and dosed introperitoneally. During the course of the experiment, tumor length and width were measured by caliper, and tumor volume calculated using the formula volume = (length x width²)^{∗}π/6.

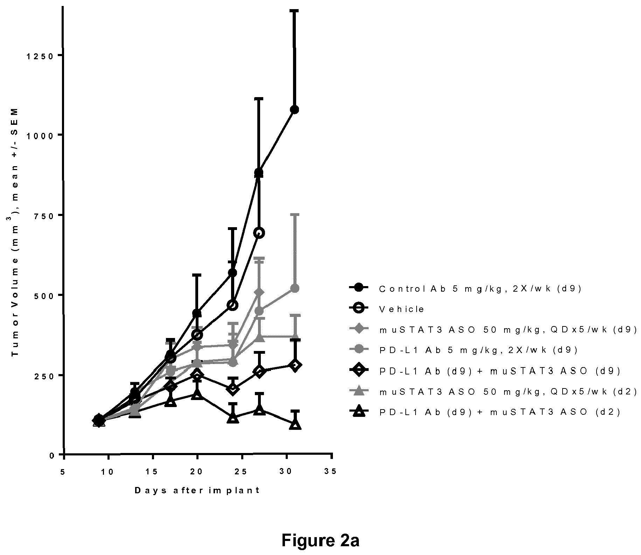

As shown in Figure 2a, treatment with the control Ab had no significant effect on tumor growth. Treatment with the mouse STAT3 ASO and anti-PD-Ll Ab as single agents had a modest effect on tumor growth. Treatment with STAT3 ASO + anti-PD-Ll Ab combination had greater antitumor activity than single agent treatments.

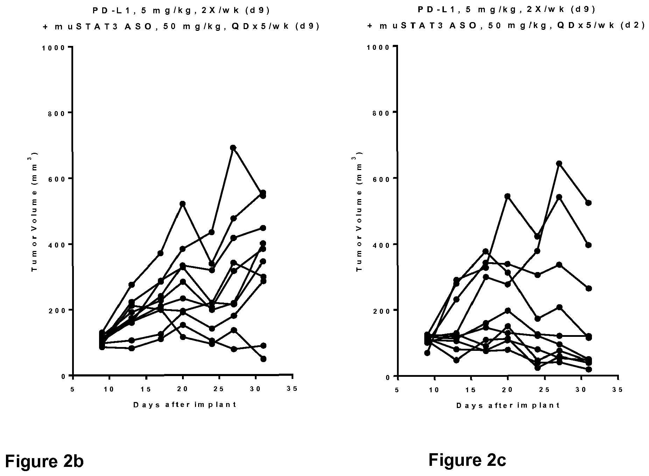

When treatment with STAT3 ASO began prior to treatment with anti-PD-Ll Ab (ASO treatment beginning day 2 and Ab treatment beginning day 9), tumor regression was observed in 6/10 mice (60%), while tumor regression was observed in only 1/10 mice (10%) when both treatments began on day 9. Figure 2a shows mean +/- SEM. Figure 2b shows individual animals for combination when dosed concurrently. Figure 2c shows individual animals for combination when predosed with ASO.

These results indicate that starting STAT3 ASO treatment prior to checkpoint inhibitor treatment provides greater antitumor activity. Thus, it seems that enabling the immune stimulatory effects of STAT3 ASO treatment to be already in progress at the time of checkpoint inhibitor treatment is beneficial.

### Example 3. Addition of mouse STAT3 ASO treatment enhances the antitumor activity of antibodies targeting PD-L1 and CTLA-4, and an OX40 ligand fusion protein, in mouse syngeneic tumor models

### Checkpoint inhibitors or immune stimulators used:

Anti-mouse CTLA-4: Clone 9D9, Mouse IgG2b isotype. Purchased from BioXCell, West Lebanon, NH
Anti-mouse PD-L1: Clone 10F.9G2, Rat IgG2b isotype. Purchased from BioXCell, West Lebanon, NH

Mouse OX40 ligand fusion protein (mIgG1FcmTF2mOX40L or OX40FP), was generated in house. The DNA and amino acid sequences are presented as SEQ ID NO: 37 and SEQ ID NO: 38, respectively.

The antitumor efficacy of mouse STAT3 ASO in combination with antibodies targeting PD-L1 or CTLA-4, or with an OX40 ligand fusion protein, were evaluated in three mouse syngeneic tumor models: CT-26 (colorectal), 4T1 (mammary) and A20 (lymphoma). Details of tumor implantation and treatment schedules for each model are in the table below. The QDx5/wk dosing schedule for the STAT3 ASO (same ASO as used in Example 1) was 5 days of treatment followed by 2 days without treatment. The 2X/wk dosing schedule for antibodies was 2 treatments/wk evenly spaced over the course of the week (e.g., Monday and Thursday, or Tuesday and Friday, etc.). ASO treatments began before randomization into treatment groups. At the time of randomization, the tumor volume in mice receiving ASO treatment was not significantly different than the tumor volume in mice that had not received ASO treatment. There were ten mice in each treatment group.

| | CT-26 | 4T1 | A20 |
|---|---|---|---|
| Implantation | 0.5 million cells, subcutaneously in right flank | 0.4 million cells in mammary fat pad | 1 million cells, subcutaneously in right flank |
| Tumor volume at randomization | Mean: 147 mm³ Range: 58-248 mm³ | Mean: 123 mm³ Range: 70-182 mm³ | Mean: 218 mm³ Range: 122-277 mm³ |
| Schedule of ASO treatment | Beginning 7 days before Ab or FP treatment, QDx5/wk for 4 weeks, dosed SC | Beginning 5 days before Ab or FP treatment, QDx7/wk for 1 week followed by QDx5/wk for 3 weeks, dosed SC | Beginning 4 days before Ab or FP treatment, QDx5/wk for 3 weeks, dosed SC |
| Randomization (days after implant | 7 | 5 | 13 |
| Dosing schedule for PD-L1 and CTLA-4 antibodies (beginning one day after randomization | 2X/wk for 3 weeks, dosed IP | 2X/wk for 1 week, dosed IP | 2X/wk for 2 weeks, dosed IP |
| Dosing schedule for OX40 fusion protein (beginning one day after randomization) | 2X/wk for 1 week, dosed IP | 2X/wk for 1 week, dosed IP | 2X/wk for 1 week, dosed IP |

The mouse STAT3 ASO was formulated in PBS and dosed subcutaneously. The Abs and FP were also formulated in PBS, and dosed introperitoneally. During the course of the experiment, tumor length and width were measured by caliper, and tumor volume calculated using the formula volume = (length x width²)^{∗} π/6.

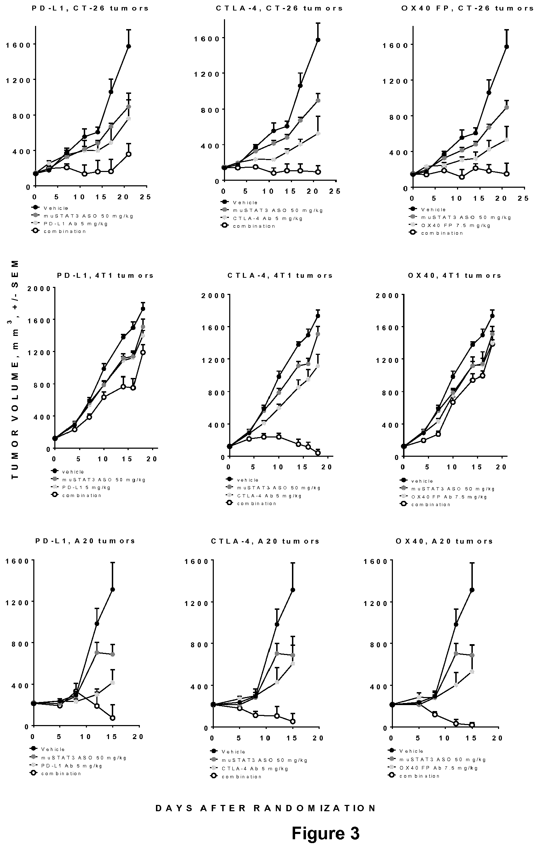

Results are shown in Figure 3. At the doses and schedules administered in this experiment, the anti-PD-Ll and anti-CTLA-4 Abs, OX40 ligand FP, and mouse STAT3 targeted ASO had weak to modest single agent antitumor activity. The addition of mouse STAT3 ASO treatment significantly enhanced the antitumor activity of antibody and FP treatment in many cases. In certain instances the effect appeared synergistic. Addition of mouse STAT3 ASO to PD-L1 Ab, CTLA-4 Ab, or OX40 ligand FP led to mean tumor stasis or regression in the CT-26 and A20 models. In the 4T1 model, the combination of mouse STAT3 ASO plus CTLA-4 antibody led to tumor regressions. In addition, the combination treatments led to long term complete responses (no measurable tumor several weeks after the end of treatment) in some individual mice, including 30% (3/10) of mice with the mouse STAT3 ASO + CTLA-4 Ab combination in the CT-26, 4T1 and A20 models; 20% (2/10) with the PD-L1 combination in the A20 model, and 50% (5/10) with the OX40 combination in the A20 model.

The enhanced antitumor activity of combination treatment vs. single agents in these models indicates the potential for treatment with a STAT3 targeted antisense molecule to enhance the activity of the checkpoint inhibitor or immune stimulators used, including agents targeting PD-L1, CTLA-4, and OX40, in multiple tumor types. The combination activity is consistent with a mechanism whereby treatment with the STAT3 ASO results in an increase in antitumor immune infiltrates in the tumor (Example 1), which enables enhanced inhibition of tumor growth when immune checkpoints are blocked by treatment with therapeutics targeting PD-L1 and CTLA-4, or when immune stimulators such as OX40 agonists are used.

### Example 4. No antitumor activity observed with a control ASO and control antibodies in mice bearing CT-26 tumors

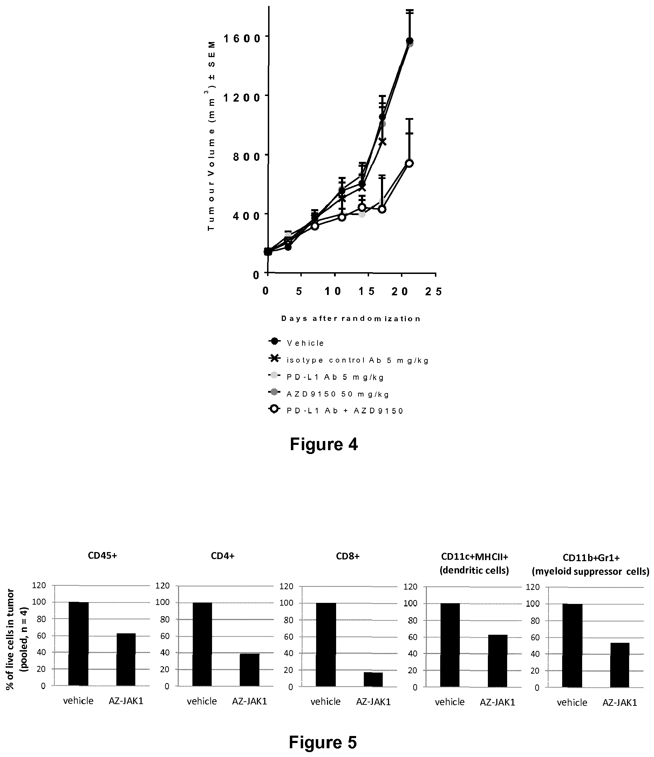

The CT-26 experiment in Example 3 included treatment with a control ASO (AZD9150) and an isotype control antibody (rat IgG2b, product LTF-2, purchased from BioXCell, West Lebanon NH) as negative controls. The human STAT3 targeted ASO molecule AZD9150 can be used as a control in murine tumor models, as it does not cross react with mouse STAT3 sequences. As shown in Figure 4, and in contrast to the activity with mouse targeted STAT3 ASO described in Examples 2 and 3, treatment with the control ASO resulted in no enhancement of the activity of the anti-PD-L1 antibody. These results are consistent with the activities of the mouse STAT3 ASO and anti-PD-L1 antibodies being due to the on target activities of these agents.

### Example 5. STAT3 inhibition using a small molecule JAK1 inhibitor suppresses the immune system

CT-26 mouse colon tumor cells (5 x 10⁵ / mouse) were implanted subcutaneously in female BALB/c mice. Ten days after implant, the mice were randomized into groups of 4, with an average tumor volume of 122mm³, and treated with either a JAK1 selective small molecule inhibitor (AZ-3; Woessner et al., Proc. Am. Assoc. Cancer Res. 54: 931, 2013), or vehicle. AZ-3 is herein after referred to as AZ-JAK1. AZ-JAK1 was formulated in water and dosed orally at 100 mg/kg, BID, for 11 days. On day 11, tumors were harvested and processed for flow cytometry analysis. Tumors from each group were pooled prior to analysis, to enable detection of low percentage cell populations by flow cytometry. CD45+ leukocytes, CD4+ and CD8+ T-cells, CD11c+MHCII+ dendritic cells, and CD11b+Gr1+ myeloid suppressor cells were quantified.

Treatment with the JAK1 selective inhibitor resulted in a decrease in T-cells, dendritic cells, and myeloid suppressor cells (Figure 5), indicating that the treatment was broadly immune suppressive, in contrast to the treatment with STAT3 ASO (Figure 1). The data indicate that selective inhibition of STAT3 with a STAT3 targeted antisense oligonucletide can enhance antitumor immune response, while inhibition of JAK1, which inhibits signaling through several STATs including STAT3 but also STATs 1, 4 and 6, is broadly immune suppressive.

### Example 6. JAK1 inhibition antagonizes the anti-tumor activity of anti-PD-Ll and anti-CTLA-4 Abs in mice bearing established CT-26 tumors

CT-26 mouse colon tumor cells (5 x 10⁵ / mouse) were implanted subcutaneously in female BALB/c mice. On day 14 after implantation, the mice were randomized into groups of 10 (based on tumor volume), and treated with either PBS vehicle, a JAK1 selective small molecule (AZ-JAK1) at 100 mg/kg BID, anti-PD-Ll antibody (clone 10F.9G2) at 2.5 mg/kg twice a week, anti-CTLA-4 antibody (clone 9D9) at 1 mg/kg twice a week, or combinations of AZ-JAK1 + anti-PD-Ll Ab or AZ-JAK1 + anti-CTLA-4 Ab. AZ-JAK1 was formulated in water and dosed orally. The antibodies were formulated in PBS, and dosed intraperitoneally. The average tumor size at the start of treatment was ∼140mm³. During the course of the experiment, tumor length and width were measured by caliper, and tumor volume calculated using the formula volume = (length x width²)^{∗}π/6.

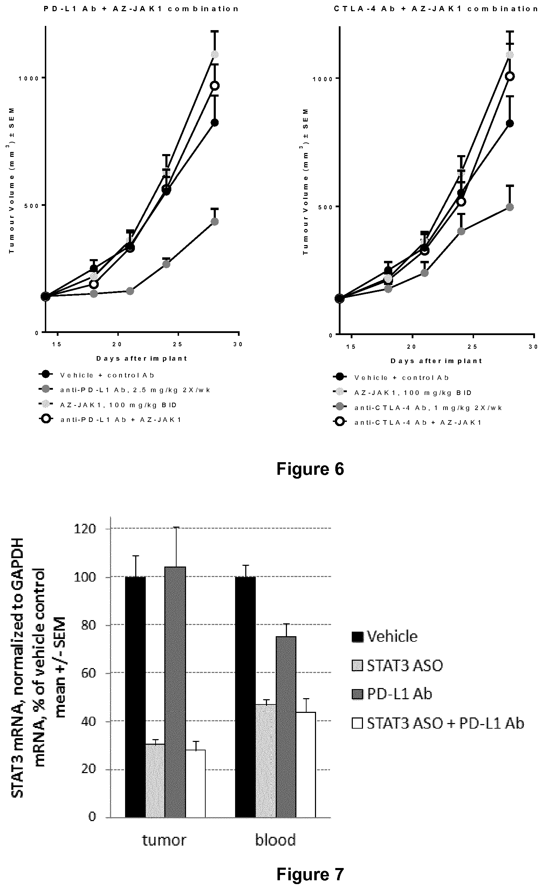

Results are shown in Figure 6. Treatment with the anti-PD-Ll and anti-CTLA-4 antibodies as a single agents resulted in inhibition of tumor growth. Treatment with AZ-JAK1 resulted in a modest increase in average tumor growth rate relative to vehicle control. Addition of AZ-JAK1 to anti-PD-Ll Ab or to anti-CTLA-4 Ab antagonized the antitumor activity of the antibodies, resulting in a tumor growth rate similar to that of single agent AZ-JAK1. All treatments were well tolerated, with no significant outward signs over the course of treatment. Average mouse weight at the end of treatment was greater than the weight at the start of treatment in all groups.

The enhancement of anti-PD-Ll or anti-CTLA-4 Ab activity by addition of a STAT3 ASO (Figures 2 and 3), vs. antagonism of antibody activity by addition of the JAK1 inhibitor (Figure 6), indicates a differential effect that is dependent on the mechanism of STAT3 inhibition (selective reduction of STAT3 protein by STAT3 ASO treatment, vs. inhibition of signaling mediated by multiple STATs, including STATs 1, 3, 4 and 6, by JAK1 inhibitor treatment).

The immune stimulation and anti-tumor activity of STAT3 ASO treatment (Figures 1 - 3), vs. the immune suppression and antagonism of anti-PD-Ll and anti-CTLA-4 Ab activities with AZ-JAK1 (Figures 5 and 6), indicate that the combination activity of STAT3 ASO + anti-PD-Ll Ab treatment is dependent on a direct, selective STAT3 inhibition, and cannot be replicated (and is in fact antagonised) by other approaches to inhibition of JAK/STAT signaling such as JAK1 inhibition.

### Example 7. Data showing STAT3 knockdown in CT-26 tumors and blood with STAT3 ASO treatment

To confirm knockdown of STAT3 mRNA by the STAT3 targeted ASO, mice bearing subcutaneous CT-26 tumors were treated with vehicle, mouse STAT3 ASO (same ASO as used in Example 1; QD, 50 mg/kg for 3.5 weeks), anti-PD-Ll Ab (anti-mouse PD-L1: Clone 10F.9G2, Rat IgG2b isotype; 2X/wk, 2.5 mg/kg for 3.5 weeks) or a combination of STAT3 ASO plus antiPD-L1 Ab, and blood and tumor samples collected at the end of the experiment and analyzed for the level of STAT3 mRNA. Samples from 4 mice in each treatment group were collected and processed for qRT-PCR analysis. Blood was collected in Paxgene reagent and RNA prepared according to kit directions (Qiagen, catalog number 762164). Tumors were collected in RNAlater (Qiagen) and processed to RNA according to kit directions (Qiagen, catalog number 74104) for qRT-PCR analysis. The STAT3 mRNA level measured by qRT-PCR was normalized to the amount of GAPDH mRNA in each sample. Primer/probe sets were purchased from ABI: catalog #4331182 for mouse STAT3, catalog #4352339E for mouse GAPDH.

STAT3 mRNA knockdown in blood and tumor, after STAT3 ASO treatment was 53% and 70%, respectively (Figure 7). STAT3 mRNA knockdown mice treated with PD-L1 Ab + STAT3 ASO was not significantly different than in mice treated with STAT3 ASO alone.

### Example 8. Gene expression changes in DLBCL patients treated with AZD9150

Tumor biopsies were collected in a Phase I clinical trial from cancer patients treated with AZD9150 before treatment started and at 4-6 weeks after treatment commenced. Patients received three loading doses 2 or 3 mg/kg AZD9150 during the first week on days 1, 3, and 5, followed by weekly doses thereafter. Nine of the sets of biopsies were from patients with Diffuse Large B Cell Lymphoma, two were from patients with follicular lymphoma, and one was from a patient with non-small cell lung cancer. The biopsies were formalin fixed and paraffin embedded and sectioned. RNA was isolated from six five-micron sections of each biopsy and subjected to Nanostring gene expression profiling analysis using the nCounter Human Immunology Codeset representing 594 immune-related genes. Raw data were calibrated according to Veldman-Jones et al., (Cancer Research. 75:2587, 2015) and analyzed for changes on-treatment compared to the baseline pretreatment samples. A heatmap representing unsupervised clustering of the largest and most statistically relevant gene expression changes in DLBCL was generated. Among the AZD9150-upregulated genes are five of six genes comprising an "interferon gamma gene signature" reported to correlate with clinical benefit of pembrolizumab, a PD1-targeting therapeutic antibody (Plimack et al, J Clin Oncol 33, 2015 (suppl; abstr 4502). These five AZD9150-upregulated "interferon gamma gene signature" genes were: STAT1, IFN-gamma, CXCL9, CXCL10, and IDO. These data indicate immune modulation in the tumor microenvironment after AZD9150 treatment of patients, and suggest that AZD9150 treatment may make patients' tumors more responsive to PD(L)1 axis therapy.

### Example 9. Prospective combination trials

Clinical trials of the MEDI4736 and AZD9150 combination are planned in squamous cell carcinoma of the head or neck (SCCHN) and diffuse large B cell lymphoma (DLBCL) to establish safe doses for the combination and test for superior clinical response rates vs. either therapy alone. The indications were chosen based on demonstration of clinical responses in previous clinical trials to monotherapy MEDI4736 in SCCHN and to AZD9150 or anti-PDl therapy in DLBCL.

AZD9150 will be formulated in saline and administered via infusion on days 1, 3, 5 of treatment and weekly thereafter. MEDI4736 will be formulated in saline and administered via intravenous infusion once every two weeks, either starting at the same time as first AZD9150 administration, or after 1, 2, or more weeks of AZD9150 administration, to allow for AZD9150 loading dose phase.

### Example 9a: Squamous Cell Carcinoma of the Head or Neck (SCCHN) trial

Subjects in this study are required to be 18 years of age or older and have histologically- or cytologically-confirmed SCCHN with at least one measurable lesion according to Response Evaluation Criteria in Solid Tumors (RECIST) guidelines v1.1.

### Design of the SCCHN trial

The study is an open-label Phase 1/2 study of the combination of anti-PD-Ll antibody (MEDI4736) and STAT3 ASO (AZD9150) vs. AZD9150 alone to establish doses, safety, and superior efficacy of the combination vs. monotherapy using RECIST criteria. Phase 1a: To establish safety and the Phase 1b doses, a small cohort of subjects (e.g. 6) will receive combinations of up to 3 mg/kg AZD9150 (starting at 3 mg/kg and lowering if there are safety issues) and 3 or 10 mg/kg MEDI4736.

Phase 1b: Up to fifty patients will receive the doses of AZD9150 + MEDI4736 established in Phase 1a and up to fifty patients will receive AZD9150 alone at the same dose as that used in the combination. The MEDI4736 monotherapy response rate from a previous clinical trial of MEDI4736 in SCCHN will be used as another comparator to the combination therapy.

Baseline levels of PDL-1 tumor expression will be obtained for all subjects to correlate to clinical responses. In addition, some circulating leukocyte populations (e.g. myeloid derived suppressor cells, MDSCs) will be monitored by immunophenotyping to assess correlation to clinical response.

Success is defined as a superior response rate or survival benefit for AZD9150 and MEDI4736 combination vs. either drug at its recommended monotherapy dose in the entire patient population or a biomarker-defined subset.

### Example 9b: Diffuse Large B Cell Lymphoma (DLBCL) trial

Subjects in this study are required to be 18 years of age or older and have histologically- or cytologically-confirmed DLBCL with at least one measurable lesion according to the International Working Group (IWG) criteria. Eligible patients must have failed Autologous Stem Cell Transplant (ASCT) or have had at least two prior multi-agent chemotherapy regimens and not be candidates for ASCT.

### Design of the DLBCL trial

The study is an open-label Phase 1/2 study of the combination of anti-PD-Ll antibody (MEDI4736) and AZD9150 vs. MEDI4736 alone to establish doses, safety, and superior efficacy of the combination vs. monotherapy using IWG criteria.
Phase 1a: To establish safety and the Phase 1b doses, a small cohort of subjects (e.g. 6) will receive combinations of up to 3 mg/kg AZD9150 (starting at 3 mg/kg and lowering if there are safety issues) and 3 or 10 mg/kg MEDI4736.

Phase 1b: Up to fifty patients will receive the doses of AZD9150 + MEDI4736 established in Phase 1a and up to fifty patients will receive MEDI4736 alone at the same dose as that used in the combination. The AZD9150 monotherapy response rate from a previous clinical trial of AZD9150 in DLBCL will be used as another comparator to the combination therapy.

Baseline levels of PDL-1 tumor expression will be obtained for all subjects to correlate to clinical responses. In addition, some circulating leukocyte populations (e.g. myeloid derived suppressor cells, MDSCs) will be monitored by immunophenotyping to assess correlation to clinical response.

Success is defined as a superior response rate or survival benefit for AZD9150 and MEDI4736 combination vs. either drug at its recommended monotherapy dose in the entire patient population or a biomarker-defined subset.

### Other Embodiments

From the foregoing description, it will be apparent that variations and modifications may be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The following further aspects are provided:
1. A method of treatment comprising administering: (i) an anti-PD-Ll antibody or an antigen-binding fragment thereof; and (ii) an antisense compound targeted to STAT3; to a patient in need thereof.
2. The method of aspect 1, wherein the anti-PD-Ll antibody is selected from: MEDI4736, MPDL3280A, BMS936559, 2.7A4, AMP-714 and MDX-1105.
3. The method of aspect 2, wherein the anti-PD-Ll antibody, is MEDI4736.
4. The method of aspect 1, wherein the antisense compound targeted to STAT3 does not inhibit STAT1, STAT4, or STAT6.
5. The method of aspect 1, wherein the antisense compound targeted to STAT3 is an antisense oligonucleotide.
6. The method of aspect 1 or aspect 5, wherein the antisense compound targeted to STAT3 is AZD9150.
7. The method of aspect 1, wherein the patient has cancer.
8. The method of aspect 7, wherein the cancer is selected from: lung cancer; including non small cell lung cancer (NSCLC); breast cancer, including triple negative; ovarian cancer, including serous; pancreatic cancer; colorectal cancer; hepatocellular carcinoma (HCC); head and neck cancer, including head and neck squamous cell carcinoma (HNSCC); and, lymphoma, including diffuse large B-cell carcinoma (DLBCL).
9. The method of aspect 8, wherein the cancer cells express PD-L1.
10. The method of aspect 1, wherein the patient in need thereof was identified as having a cancer that is PD-L1 positive.
11. The method according to any of the preceding aspect wherein the anti-PD-Ll antibody is MEDI4736 and the antisense compound targeted to STAT3 is AZD9150.
12. The method according to aspect 11, wherein between about 1 mg/kg and 20 mg/kg MEDI4736 or an antigen-binding fragment thereof, and between about 1 mg/kg and 10 mg/kg AZD9150 is administered to a patient in need thereof.
13. The method of aspect 12, wherein the treatment is administered every week, every 2 weeks, every 3 weeks, or every 4 weeks.
14. The method of any one of aspects 1-13, wherein about 1 mg/kg MEDI4736 or an antigen-binding fragment thereof, and about 3 mg/kg AZD9150 is administered per dose.
15. The method of any one of aspects 1-14, wherein the method results in an increase in progression free survival and/or overall survival as compared to the administration of either the MEDI4736 or the AZD9150 alone.
16. The method of any one of aspects 2-15, wherein the administration of MEDI4736 or an antigen-binding fragment thereof, is by intravenous infusion.
17. The method of any one of aspects 4-16, wherein the administration of AZD9150 is by intravenous infusion.
18. The method of any one of aspects 2-15, wherein AZD9150 and MEDI4736, or an antigen-binding fragment thereof, are administered concurrently or at different times.
19. A kit for treating cancer, the kit comprising MEDI4736 or an antigen-binding fragment thereof, and AZD9150.
20. A pharmaceutical composition which comprises MEDI4736 or an antigen-binding fragment thereof, and AZD9150 in association with a pharmaceutically acceptable diluent or carrier.
21. The pharmaceutical composition of aspect 20, wherein the pharmaceutical composition is formulated to provide a dose of about 1mg/kg to about 20mg/kg of MEDI4736 or an antigen-binding fragment thereof, and a dose of about 1mg/kg to about 10mg/kg of AZD9150.
22. The pharmaceutical composition of aspect 21, wherein the pharmaceutical composition is formulated to provide a dose of about 1mg/kg of MEDI4736 or an antigen-binding fragment thereof, and a dose of about 3mg/kg of AZD9150.
23. A method for modulating immune infiltrate cells in a warm-blooded animal such as a human, which comprises administering to said animal an effective amount of MEDI4736 or an antigen binding fragment thereof, before, after or simultaneously with an effective amount of AZD9150.

## Claims

1. A combination comprising an immune checkpoint inhibitor and an antisense compound targeted to STAT3 for use in the treatment of cancer.

2. A combination for use according to claim 1, wherein the immune checkpoint inhibitor is selected from: an anti-PD-Ll antibody or an antigen-binding fragment thereof; an anti-PDl antibody or an antigen-binding fragment thereof; an anti-CTLA-4 antibody or an antigen-binding fragment thereof; and, an OX-40 agonist.

3. A combination for use according to claim 1 or claim 2, wherein the immune checkpoint inhibitor is selected from: MEDI4736, MPDL3280A, 2.7A4, AMP-714, MDX-1105, nivolumab, pembrolizumab, pidilizumab, BMS936559, MPDL3280A, tremelimumab, ipilimumab and OX40L FP.

4. A combination for use according to any one of claims 1 - 3, wherein the immune checkpoint inhibitor is MEDI4736.

5. A combination for use according to any of the previous claims wherein the antisense compound targeted to STAT3 does not inhibit STAT1, STAT4, or STAT6.

6. A combination for use according to any of the previous claims, wherein the antisense compound targeted to STAT3 is an antisense oligonucleotide.

7. A combination for use according to any of the previous claims, wherein the antisense compound targeted to STAT3 is AZD9150.

8. A combination for use according to any of the previous claims, wherein the cancer is selected from: lung cancer, including non small cell lung cancer (NSCLC), pancreatic cancer, colorectal cancer, hepatocellular carcinoma (HCC), head and neck cancer, including head and neck squamous cell carcinoma (HNSCC), and lymphoma, including diffuse large B-cell carcinoma (DLBCL).

9. A combination for use according to claim 8, wherein the cancer cells express PD-L1.

10. A combination for use according to any of the previous claims, wherein the cancer is PD-L1 positive.

11. A combination for use according to any of the previous claims wherein the immune checkpoint inhibitor is MEDI4736 and the antisense compound targeted to STAT3 is AZD9150.

12. A combination for use according to claim 11, wherein between about 1 mg/kg and 20 mg/kg MEDI4736, and between about 1 mg/kg and 10 mg/kg AZD9150 is administered to a patient in need thereof.

13. A combination for use according to claim 12, wherein the treatment is administered every week, every 2 weeks, every 3 weeks, or every 4 weeks.

14. A combination for use according to any of the previous claims wherein the antisense compound targeted to STAT3 is administered to the patient before the immune checkpoint inhibitor.

15. A combination for use according to claim 14, wherein the antisense compound targeted to STAT3 is administered to the patient at least one day before the immune checkpoint inhibitor is administered to the patient.

16. A combination for use according to any one of claims 1 - 15, wherein the antisense compound targeted to STAT3 is AZD9150 and the immune checkpoint inhibitor is MED14736, and wherein AZD9150 and MEDI4736 are administered concurrently or at different times.

17. A combination for use according to any of the claims 1 - 16, comprising administering (i) two immune checkpoint inhibitors and (ii) an antisense compound targeted to STAT3 to a patient in need thereof; wherein the two immune checkpoint inhibitors are MEDI4736 and tremelimumab, and the antisense compound targeted to STAT3 is AZD9150

18. A pharmaceutical composition which comprises an immune checkpoint inhibitor and an antisense compound targeted to STAT3 in association with a pharmaceutically acceptable diluent or carrier for use in the treatment of cancer.
